(19) Europäisches Patentamt
European Patent Office
Office européen des brevets

(11) EP 4 751 739 A1

(12) **EUROPEAN PATENT APPLICATION**
published in accordance with Art. 153(4) EPC

(43) Date of publication:
**03.06.2026 Bulletin 2026/23**

(21) Application number: **24844837.5**

(22) Date of filing: **25.07.2024**

(51) International Patent Classification (IPC):
***A61K 47/68*** *(2017.01)* ***A61K 31/506*** *(2006.01)*
***A61P 35/00*** *(2006.01)*

(52) Cooperative Patent Classification (CPC):
**A61K 31/506; A61K 47/68; A61P 35/00**

(86) International application number:
**PCT/CN2024/107536**

(87) International publication number:
**WO 2025/021151 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.07.2023 CN 202310916920**

(71) Applicants:
- **Jiangsu Hengrui Pharmaceuticals Co., Ltd.**
  **Lianyungang, Jiangsu 222047 (CN)**
- **Shanghai Shengdi Pharmaceutical Co., Ltd**
  **Shanghai 201210 (CN)**

(72) Inventors:
- **TIAN, Yunan**
  **Shanghai 201210 (CN)**
- **SUN, Xing**
  **Shanghai 201210 (CN)**
- **SU, Lu**
  **Shanghai 201210 (CN)**
- **YUAN, Jimin**
  **Shanghai 201210 (CN)**
- **LIAO, Cheng**
  **Lianyungang, Jiangsu 222047 (CN)**

(74) Representative: **Dragotti & Associati S.P.A.**
**Via Nino Bixio, 7**
**20129 Milano (MI) (IT)**

Remarks:
The complete document including Reference Table(s) and the Sequence Listing(s) can be downloaded from the EPO website

(54) **ANTIBODY-DRUG CONJUGATE TARGETING C-MET AND MEDICAL USE THEREOF**

(57) An antibody-drug conjugate targeting c-Met and the medical use thereof. The anti-c-Met antibody-drug conjugate has the structure as shown in formula (I): AB-(L-Y-D)n(I), wherein Ab is an anti-c-Met antibody, and L, Y and D are as defined in the description.

EP 4 751 739 A1

## Description

The present disclosure claims priority to Patent Application No. CN202310916920.7, filed on July 25, 2023.

## TECHNICAL FIELD

The present disclosure relates to an anti-c-Met antibody-drug conjugate, a preparation method therefor, a pharmaceutical composition comprising same, and use thereof in the manufacture of a medicament for treating a c-Met-mediated disease or disorder, particularly in the manufacture of an anticancer medicament.

## BACKGROUND

In recent years, studies in molecular biology and oncopharmacology have shown that cell signaling pathways associated with protein tyrosine kinases (PTKs) play a crucial role in the development and progression of tumors. Over 50% of proto-oncogene and oncogene products have tyrosine kinase activity. The c-Met proto-oncogene belongs to the Ron subfamily in the PTKs family, and its encoded protein, c-Met, is a high-affinity receptor for hepatocyte growth factor/scatter factor (HGF/SF). The HGF/c-Met signaling pathway is closely related to angiogenesis and tumor growth, and its persistent activation is a significant cause of the canceration of histiocytes or the hyperproliferation of cancer cells. Inhibiting this pathway has become a new approach to targeted cancer therapy.

The c-Met proto-oncogene is located on the long arm of human chromosome 7 (7q31) and is more than 120 kb in size. It encodes a precursor to the c-Met protein with a molecular weight of about 150 kD, which undergoes local glycosylation to produce a 170 kD glycoprotein. The glycoprotein is further cleaved into an $\alpha$ subunit (50 kDa) and a $\beta$ subunit (140 kDa), which are linked by disulfide bonds to form a mature c-Met protein receptor. This heterodimer comprises two chains. The $\beta$ chain has an extracellular region, a transmembrane region (also known as a membrane-spanning fragment), and an intracellular region (containing an intracellular tyrosine kinase binding site). The $\alpha$ chain has only an extracellular portion, and it is highly glycosylated and is attached to the $\beta$ chain through disulfide bonds. The extracellular regions of the two subunits are the recognition sites for the corresponding ligands, and the intracellular region has tyrosine kinase activity.

There are three mechanisms of c-Met activation: one dependent on HGF, one independent of HGF, and one through other membrane pathways, such as the CD44, adhesin, and RON signaling pathways through hyaluronic acid membrane surface receptors, etc. The most common of these is the HGF-dependent mechanism of activation. The N-terminus of HGF binds to c-Met, promoting dimerization and autophosphorylation of Tyr1234 and Tyr1235 on the $\beta$ chain. The phosphorylation of Tyr1349 and Tyr1356 near the C-terminus creates binding sites for multiple linker proteins, which induce the activation of downstream signals mediated by Pi3K/Akt, Ras/MAPK, c-Src, and STAT3/5, leading to various cellular responses such as cell survival and motility (closely related to the Pi3K/Akt pathway), tumor metastasis, and cell proliferation (mainly mediated by Ras/MAPK). Additionally, c-Met cross-talks with other membrane receptors, and such a cross-talk is known to promote tumor development and metastasis. Since c-Met is a convergence point for many pathways leading to tumor development and metastasis, targeting c-Met can relatively easily interfere with many pathways simultaneously, which makes c-Met a promising target for therapy against tumorigenesis and metastasis. Antibody-drug conjugates (ADC) are formed by linking monoclonal antibodies or antibody fragments and cytotoxins with biological activity by stable chemical linker compounds, so that the specificity of the antibodies to bind to tumor cell-specific or highly expressed antigens and the high efficiency of the cytotoxins are combined to prevent toxic and side effects on normal cells. This means that antibody-drug conjugates can bind to tumor cells precisely and reduce the impact on normal cells compared to conventional chemotherapeutic drugs.

Reported c-Met-targeting ADCs include Telisotuzumab Vedotin (ABBV-399, AbbVie, WO2017201204) and TR1801-ADC (Tanabe, Gymnopoulos, Marco, et al. "TR1801-ADC: a highly potent cMet antibody-drug conjugate with high activity in patient-derived xenograft models of solid tumors." Molecular oncology 14.1 (2020): 54-68). Telisotuzumab Vedotin is formed by linking the anti-c-Met monoclonal antibody ABT-700 and the tubulin inhibitor MMAE by a cleavable linker unit. TR1801-ADC is a conjugate of an anti-c-Met antibody and a pyrrolobenzodiazepine dimer (PBD). While there are currently some c-Met-targeting ADCs, none have been approved for commercial availability yet. Currently, only Telisotuzumab Vedotin is in phase III clinical trials, with others still in early clinical or preclinical stages where their safety and effectiveness remain to be explored. Therefore, there is still an urgent clinical need to develop c-Met-targeting ADC drugs with high safety and good effectiveness.

## SUMMARY

The present disclosure provides an anti-c-Met antibody-drug conjugate or a pharmaceutically acceptable salt thereof and pharmaceutical use thereof.

Antibody-Drug Conjugate

The present disclosure provides a c-Met-targeting antibody-drug conjugate or a pharmaceutically acceptable salt thereof, having a structure represented by formula (I):

Ab-(L-Y-D)n formula (I)

wherein:

Ab is an anti-c-Met antibody comprising a heavy chain variable region (VH) and a light chain variable region (VL), wherein the VH comprises a HCDR1, a HCDR2, and a HCDR3 from the amino acid sequence set forth in any one of SEQ ID NOs: 10-12, and the VL comprises a LCDR1, a LCDR2, and a LCDR3 from the amino acid sequence set forth in any one of SEQ ID NOs: 13-15;
the CDRs are defined according to the Kabat, IMGT, Chothia, AbM, or Contact numbering scheme;
-L- is a linker unit that is $-L^1-L^2-L^3-L^4-$;
$L^1$ is -(succinimid-3-yl-*N*)-W-C(O)-, $-CH_2-C(O)-NR^3-W-C(O)-$, or -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$, and a chemical bond, wherein $p^1$ is an integer from 1 to 20;
$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;
$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;
$R^3$, $R^4$, and $R^5$ are identical or different and are each independently selected from the group consisting of hydrogen, alkyl, haloalkyl, deuterated alkyl, and hydroxyalkyl;
$R^6$ and $R^7$ are identical or different and are each independently selected from the group consisting of hydrogen, halogen, alkyl, haloalkyl, deuterated alkyl, and hydroxyalkyl;
Y is selected from the group consisting of $-O-(CR^aR^b)_m-CR^1R^2-C(O)-$, $-O-CR^1R^2-(CR^aR^b)_m-$, $-O-CR^1R^2-$, $-NH-(CR^aR^b)_m-CR^1R^2-C(O)-$, and $-S-(CR^aR^b)_m-CR^1R^2-C(O)-$;
$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl, and heterocyclyl; or $R^a$ and $R^b$, together with the carbon atom to which they are attached, form cycloalkyl or heterocyclyl; $R^1$ is selected from the group consisting of hydrogen, halogen, haloalkyl, deuterated alkyl, cycloalkyl, cycloalkylalkyl, alkoxyalkyl, heterocyclyl, aryl, and heteroaryl;
$R^2$ is selected from the group consisting of hydrogen, halogen, haloalkyl, deuterated alkyl, cycloalkyl, cycloalkylalkyl, alkoxyalkyl, heterocyclyl, aryl, and heteroaryl;
or $R^1$ and $R^2$, together with the carbon atom to which they are attached, form cycloalkyl or heterocyclyl;
or $R^a$ and $R^2$, together with the carbon atoms to which they are attached, form cycloalkyl or heterocyclyl;
m is an integer from 0 to 4;
n is 1 to 10, and n is an integer or decimal;
D is a camptothecin drug.

In some embodiments, D is exatecan or a derivative thereof.

In some embodiments, $L^1$ is linked to Ab, and $L^4$ is linked to Y.

In some embodiments, the aforementioned antibody-drug conjugate represented by formula (I) or the pharmaceutically acceptable salt thereof is provided, wherein the anti-c-Met antibody comprises a heavy chain variable region (VH) and a light chain variable region (VL); the VH and the VL are selected from the group consisting of any one of the following:

(1) the VH comprises a HCDR1, a HCDR2, and a HCDR3 set forth in SEQ ID NOs: 1-3, and the VL comprises a LCDR1, a LCDR2, and a LCDR3 set forth in SEQ ID NOs: 22, 5, and 6; for example, the amino acid sequence set forth in SEQ ID NO: 22 is $RAX_1KSVSTSTYNYLH$, and $X_1$ is selected from the group consisting of N and D;

Table 1. CDR sequences

| Name | Amino acid sequence | No. |
|---|---|---|
| HCDR1 | NYGVH | SEQ ID NO: 1 |
| HCDR2 | VIWSGGSTNYAAAFVS | SEQ ID NO: 2 |
| HCDR3 | NHDNPYNYAMDY | SEQ ID NO: 3 |
| LCDR1 | RANKSVSTSTYNYLH | SEQ ID NO: 4 |
| LCDR2 | LASNLAS | SEQ ID NO: 5 |
| LCDR3 | QHSRDLPPT | SEQ ID NO: 6 |
| LCDR1 | RADKSVSTSTYNYLH | SEQ ID NO: 7 |

(2) the VH comprises a HCDR1, a HCDR2, and a HCDR3 from the amino acid sequence set forth in any one of SEQ ID NOs: 10-12, and the VL comprises a LCDR1, a LCDR2, and a LCDR3 from the amino acid sequence set forth in any one of SEQ ID NOs: 13-15. In some embodiments, the CDRs are defined according to the Kabat, IMGT, Chothia, AbM, or Contact numbering scheme. In some specific embodiments, the CDRs are defined according to the Kabat numbering scheme.

In some embodiments, the anti-c-Met antibody comprises a VH and a VL, wherein any HCDR contained in the VH comprises 0, 1, 2, 3, 4, or 5 amino acid mutations compared to any one of the aforementioned HCDRs, and/or any LCDR contained in the VL comprises 0, 1, 2, 3, 4, or 5 amino acid mutations compared to any one of the aforementioned LCDRs. In some specific embodiments, the above amino acid mutations are conservative replacements, substitutions, or modifications, and/or deletions or additions that do not affect function.

In some embodiments, the antibody-drug conjugate represented by formula (I) or the pharmaceutically acceptable salt thereof according to any one of the foregoing is provided, wherein the VH of the anti-c-Met antibody comprises a HCDR1, a HCDR2, and a HCDR3 set forth in SEQ ID NOs: 1, 2, and 3, and the VL comprises a LCDR1, a LCDR2, and a LCDR3 set forth in SEQ ID NOs: 7, 5, and 6.

In some embodiments, the antibody-drug conjugate represented by formula (I) or the pharmaceutically acceptable salt thereof according to any one of the foregoing is provided, wherein the VH of the anti-c-Met antibody comprises a HCDR1, a HCDR2, and a HCDR3 set forth in SEQ ID NOs: 1, 2, and 3, and the VL comprises a LCDR1, a LCDR2, and a LCDR3 set forth in SEQ ID NOs: 4, 5, and 6.

In some embodiments, the antibody-drug conjugate represented by formula (I) or the pharmaceutically acceptable salt thereof according to any one of the foregoing is provided, wherein the anti-c-Met antibody is a murine antibody, a chimeric antibody, a humanized antibody, or a human antibody.

In some embodiments, the antibody-drug conjugate represented by formula (I) or the pharmaceutically acceptable salt thereof according to any one of the foregoing is provided, wherein the anti-c-Met antibody comprises a heavy chain variable region set forth in SEQ ID NO: 8 and a light chain variable region set forth in SEQ ID NO: 9. The anti-c-Met antibody is a murine antibody.

Heavy chain variable region:

QVQLKQSGPGLVQPSQSLSITCTVSGFSLP**NYGVH**WVRQSPGKGLEWLG**VIWSGGSTNYAAAFVS**RLRISKDNSKSQVFFEMNSLQADDTAVYYCAR**NHDNPYNYAMDY**WGQGTTVTVSS

SEQ ID NO: 8

Light chain variable region:

DIVLTQSPGSLAVYLGQRATISC**RANKSVSTSTYNYLH**WYQQKPGQPPKLLIY**LASNLAS**GVPARFSGSGSGTDFTLNIHPLEEEDAATYYC**QHSRDLPPT**FGAGTKLELKR

SEQ ID NO: 9

The amino acid residues of the VH/VL CDRs of the anti-human c-Met antibody are determined and annotated by the Kabat numbering scheme, and the CDRs are in bold type.

In some embodiments, the antibody-drug conjugate represented by formula (I) or the pharmaceutically acceptable salt thereof according to any one of the foregoing is provided, wherein the heavy chain variable region of the c-Met antibody further comprises heavy chain FRs of human IgG1, IgG2, IgG3, or IgG4 or a variant thereof.

In some embodiments, the antibody-drug conjugate represented by formula (I) or the pharmaceutically acceptable salt thereof according to any one of the foregoing is provided, wherein the heavy chain FR sequences in the heavy chain variable region of the c-Met antibody are derived from a human germline heavy chain sequence, e.g., the human germline heavy chain IGHV 3-33*01, and comprise the framework sequence of the FR1, FR2, FR3, and FR4 of the human germline heavy chain IGHV 3-33*01 or a mutated sequence thereof; for example, the mutated sequence comprises back mutations of 0-10 amino acids.

In some embodiments, the antibody-drug conjugate represented by formula (I) or the pharmaceutically acceptable salt thereof according to any one of the foregoing is provided, wherein the light chain FR sequences in the light chain variable region of the c-Met antibody are selected from the group consisting of human germline light chain sequences, e.g., the human germline light chain IGKV085 or IGKV 4-1*01, and comprise the framework sequence of the FR1, FR2, FR3, and FR4 of the human germline light chains IGKV085 and IGKV 4-1*01 or a mutated sequence thereof; for example, the mutated sequence comprises back mutations of 0-10 amino acids.

In some embodiments, the antibody-drug conjugate represented by formula (I) or the pharmaceutically acceptable salt thereof according to any one of the foregoing is provided, wherein the anti-c-Met antibody is a humanized antibody. In some embodiments, the antibody-drug conjugate represented by formula (I) or the pharmaceutically acceptable salt thereof according to any one of the foregoing is provided, wherein the anti-c-Met antibody comprises a heavy chain variable region sequence selected from the group consisting of SEQ ID NOs: 10-12 and a light chain variable region sequence selected from the group consisting of SEQ ID NOs: 13-15.

In some embodiments, the antibody-drug conjugate represented by formula (I) or the pharmaceutically acceptable salt thereof according to any one of the foregoing is provided, wherein the anti-c-Met antibody comprises a combination of a heavy chain variable region sequence and a light chain variable region sequence selected from the group consisting of any one of a) to c):

a) a heavy chain variable region sequence of SEQ ID NO: 10 or a heavy chain variable region sequence having at least 90% sequence identity thereto, and a light chain variable region sequence of SEQ ID NO: 13 or a light chain variable region sequence having at least 90% sequence identity thereto;
b) a heavy chain variable region sequence of SEQ ID NO: 11 or a heavy chain variable region sequence having at least 90% sequence identity thereto, and a light chain variable region sequence of SEQ ID NO: 14 or a light chain variable region sequence having at least 90% sequence identity thereto;
or c) a heavy chain variable region sequence of SEQ ID NO: 12 or a heavy chain variable region sequence having at least 90% sequence identity thereto, and a light chain variable region sequence of SEQ ID NO: 15 or a light chain variable region sequence having at least 90% sequence identity thereto.

In some embodiments, the antibody-drug conjugate represented by formula (I) or the pharmaceutically acceptable salt thereof according to any one of the foregoing is provided, wherein the anti-c-Met antibody comprises a heavy chain variable region sequence of SEQ ID NO: 11 and a light chain variable region sequence of SEQ ID NO: 14.

Heavy chain variable region:

Ab-9

QVTLKESGPVLVKPTETLTLTCTVSGFSLP**NYGVH**WVRQPPGKALEWLA**VIWS**
**GGSTNYAAAFVS**RLRISKDTSKSQVVFTMNNMDPVDTATYYCAR**NHDNPYN**
**YAMDY**WGQGTTVTVSS

SEQ ID NO: 10

Ab-10

QVQLVESGGGVVQPGRSLRLSCAASGFSLS**NYGVH**WVRQAPGKGLEWLA**VIWSGGSTNYAAAFVS**RLTISKDNSKNTVYLQMNSLRAEDTAVYYCAR**NHDNPYNYAMDY**WGQGTTVTVSS

SEQ ID NO: 11

Ab-11

QVQLVESGGGVVQPGRSLRLSCAASGFTLP**NYGVH**WVRQAPGKGLEWLA**VIWSGGSTNYAAAFVS**RLTISKDNSKNTVYLQMNSLRAEDTAVYYCAR**NHDNPYNYAMDY**WGQGTTVTVSS

SEQ ID NO: 12

Light chain variable region:

Ab-9

DIVLTQSPASLAVSPGQRATITC**RANKSVSTSTYNYLH**WYQQKPGQPPKLLIY**LASNLAS**GVPARFSGSGSGTDFTLTINPVEANDTANYYC**QHSRDLPPT**FGQGTKLEIKR

SEQ ID NO: 13

Ab-10

DIVLTQSPDSLAVSLGERATINC**RADKSVSTSTYNYLH**WYQQKPGQPPKLLIY**LASNLAS**GVPDRFSGSGSGTDFTLTISSLQAEDVAVYYC**QHSRDLPPT**FGQGTKLEIKR

SEQ ID NO: 14

Ab-11

DIVLTQSPDSLAVSLGERATINC**RANKSVSTSTYNYLH**WYQQKPGQPPKLLIY**LASNLAS**GVPDRFSGSGSGTDFTLTISSLQAEDVAVYYC**QHSRDLPPT**FGQGTKLEIKR

SEQ ID NO: 15.

In some embodiments, the anti-c-Met antibody further comprises a light chain constant region and/or a heavy chain constant region.

In some embodiments, the light chain constant region is derived from a κ light chain, a λ light chain, or a variant of any one of the foregoing. For example, the light chain constant region is derived from a human κ light chain, a human λ light chain, or a variant of any one of the foregoing. In some specific embodiments, the light chain constant region is derived from a human κ light chain or a variant thereof.

In some embodiments, the heavy chain constant region is derived from IgG1, IgG2, IgG3, IgG4, or a variant of any one of the foregoing. For example, the heavy chain constant region is derived from human IgG1, human IgG2, human IgG3, human IgG4, or a variant of any one of the foregoing.

In some embodiments, the anti-c-Met antibody further comprises an Fc region. In some specific embodiments, the Fc region is derived from the Fc region of human IgG1, IgG2, IgG3, or IgG4. In some specific embodiments, the Fc region is the Fc region of human IgG2.

In some embodiments, the antibody-drug conjugate represented by formula (I) or the pharmaceutically acceptable salt thereof according to any one of the foregoing is provided, wherein the c-Met antibody is a humanized antibody, wherein the heavy chain constant region of the humanized antibody comprises a constant region derived from human IgG1 or a variant thereof, human IgG2 or a variant thereof, human IgG3 or a variant thereof, or human IgG4 or a variant thereof. In some embodiments, the heavy chain constant region of the humanized antibody comprises a constant region of human IgG1 or a variant thereof, or human IgG2 or a variant thereof, or human IgG4 or a variant thereof. In some embodiments, the heavy chain constant region of the humanized antibody comprises a constant region of human IgG2 or a variant thereof.

In some embodiments, the antibody-drug conjugate represented by formula (I) or the pharmaceutically acceptable salt thereof according to any one of the foregoing is provided, wherein in the anti-c-Met antibody:

(1) the heavy chain comprises the amino acid sequence set forth in any one of SEQ ID NOs: 16, 18, and 20 or an amino acid sequence having at least 80% sequence identity thereto, and/or the light chain comprises the amino acid sequence set forth in any one of SEQ ID NOs: 17, 19, and 21 or an amino acid sequence having at least 80% sequence identity thereto;
(2) the heavy chain comprises the amino acid sequence set forth in SEQ ID NO: 16 or an amino acid sequence having at least 80% sequence identity thereto, and/or the light chain comprises the amino acid sequence set forth in SEQ ID NO: 17 or an amino acid sequence having at least 80% sequence identity thereto;
(3) the heavy chain comprises the amino acid sequence set forth in SEQ ID NO: 18 or an amino acid sequence having at least 80% sequence identity thereto, and/or the light chain comprises the amino acid sequence set forth in SEQ ID NO: 19 or an amino acid sequence having at least 80% sequence identity thereto; or
(4) the heavy chain comprises the amino acid sequence set forth in SEQ ID NO: 20 or an amino acid sequence having at least 80% sequence identity thereto, and/or the light chain comprises the amino acid sequence set forth in SEQ ID NO: 21 or an amino acid sequence having at least 80% sequence identity thereto.

In the context of the present disclosure, "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity; "at least 90% (sequence) identity" encompasses at least 90%, at least 91%, at least 92%, at least 93%, at least 94%, at least 95%, at least 96%, at least 97%, at least 98%, at least 99%, or 100% (sequence) identity.

**Ab-9 humanized antibody:**

Heavy chain:

QVTLKESGPVLVKPTETLTLTCTVSGFSLPNYGVHWVRQPPGKALEWLAVIWS
GGSTNYAAAFVSRLRISKDTSKSQVVFTMNNMDPVDTATYYCARNHDNPYNY
AMDYWGQGTTVTVSSASTKGPSVFPLAPCSRSTSESTAALGCLVKDYFPEPVTV
SWNSGALTSGVHTFPAVLQSSGLYSLSSVVTVPSSNFGTQTYTCNVDHKPSNTK
VDKTVERKCCVECPPCPAPPVAGPSVFLFPPKPKDTLMISRTPEVTCVVVDVSH
EDPEVQFNWYVDGVEVHNAKTKPREEQFNSTFRVVSVLTVVHQDWLNGKEY
KCKVSNKGLPAPIEKTISKTKGQPREPQVYTLPPSREEMTKNQVSLTCLVKGFY
PSDIAVEWESNGQPENNYKTTPPMLDSDGSFFLYSKLTVDKSRWQQGNVFSCS
VMHEALHNHYTQKSLSLSPGK

SEQ ID NO: 16

Light chain:

DIVLTQSPASLAVSPGQRATITCRANKSVSTSTYNYLHWYQQKPGQPPKLLIYL
ASNLASGVPARFSGSGSGTDFTLTINPVEANDTANYYCQHSRDLPPTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO: 17

**Ab-10 humanized antibody:**

Heavy chain:

QVQLVESGGGVVQPGRSLRLSCAASGFSLSNYGVHWVRQAPGKGLEWLAVIW
SGGSTNYAAAFVSRLTISKDNSKNTVYLQMNSLRAEDTAVYYCARNHDNPYN
YAMDYWGQGTTVTVSSASTKGPSVFPLAPCSRSTSESTAALGCLVKDYFPEPVT
VSWNSGALTSGVHTFPAVLQSSGLYSLSSVVTVPSSNFGTQTYTCNVDHKPSNT
KVDKTVERKCCVECPPCPAPPVAGPSVFLFPPKPKDTLMISRTPEVTCVVVDVS
HEDPEVQFNWYVDGVEVHNAKTKPREEQFNSTFRVVSVLTVVHQDWLNGKE
YKCKVSNKGLPAPIEKTISKTKGQPREPQVYTLPPSREEMTKNQVSLTCLVKGF
YPSDIAVEWESNGQPENNYKTTPPMLDSDGSFFLYSKLTVDKSRWQQGNVFSC
SVMHEALHNHYTQKSLSLSPGK

SEQ ID NO: 18

Light chain:

DIVLTQSPDSLAVSLGERATINCRADKSVSTSTYNYLHWYQQKPGQPPKLLIYL
ASNLASGVPDRFSGSGSGTDFTLTISSLQAEDVAVYYCQHSRDLPPTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO: 19

**Ab-11 humanized antibody:**

Heavy chain:

QVQLVESGGGVVQPGRSLRLSCAASGFTLPNYGVHWVRQAPGKGLEWLAVIW
SGGSTNYAAAFVSRLTISKDNSKNTVYLQMNSLRAEDTAVYYCARNHDNPYN
YAMDYWGQGTTVTVSSASTKGPSVFPLAPCSRSTSESTAALGCLVKDYFPEPVT
VSWNSGALTSGVHTFPAVLQSSGLYSLSSVVTVPSSNFGTQTYTCNVDHKPSNT
KVDKTVERKCCVECPPCPAPPVAGPSVFLFPPKPKDTLMISRTPEVTCVVVDVS
HEDPEVQFNWYVDGVEVHNAKTKPREEQFNSTFRVVSVLTVVHQDWLNGKE
YKCKVSNKGLPAPIEKTISKTKGQPREPQVYTLPPSREEMTKNQVSLTCLVKGF
YPSDIAVEWESNGQPENNYKTTPPMLDSDGSFFLYSKLTVDKSRWQQGNVFSC
SVMHEALHNHYTQKSLSLSPGK

SEQ ID NO: 20

Light chain:

DIVLTQSPDSLAVSLGERATINCRANKSVSTSTYNYLHWYQQKPGQPPKLLIYLA
SNLASGVPDRFSGSGSGTDFTLTISSLQAEDVAVYYCQHSRDLPPTFGQGTKLEI
KRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC

SEQ ID NO: 21.

In some embodiments, the antibody-drug conjugate represented by formula (I) or the pharmaceutically acceptable salt thereof according to any one of the foregoing is provided, wherein the anti-c-Met antibody comprises a heavy chain sequence set forth in SEQ ID NO: 18 and a light chain sequence set forth in SEQ ID NO: 19.

In some embodiments, the anti-c-Met antibody encompasses antigen-binding fragments. In some embodiments, the antigen-binding fragments include, but are not limited to, Fab, Fv, sFv, Fab', $F(ab')_2$, linear antibodies, single-chain antibodies, scFv, sdAb, sdFv, nanobodies, peptibodies, and domain antibodies. For example, the antigen-binding fragments are scFv, Fv, Fab, or Fab' fragments. In some embodiments, the anti-c-Met antibody includes monospecific antibodies and multispecific antibodies (e.g., bi-, tri-, and tetra-specific antibodies). In some embodiments, the anti-c-Met antibody includes diabodies, triabodies and tetrabodies, tandem di-scFv, tandem tri-scFv, etc.

In some embodiments, the anti-c-Met antibody in the antibody-drug conjugate binds to or competes for binding to the same c-Met epitope with any one of the aforementioned anti-c-Met antibodies.

In some embodiments, the anti-c-Met antibody in the antibody-drug conjugate blocks the binding of any one of the aforementioned anti-c-Met antibodies to c-Met.

In some embodiments, the binding of the anti-c-Met antibody in the antibody-drug conjugate to c-Met is blocked by any one of the aforementioned anti-c-Met antibodies. In some embodiments, the antibody-drug conjugate represented by formula (I) or the pharmaceutically acceptable salt thereof according to any one of the foregoing is provided, wherein:

Y is $-O-(CR^aR^b)_m-CR^1R^2-C(O)-$;
$R^a$ and $R^b$ are identical or different and are each independently selected from the group consisting of hydrogen, deuterium, halogen, and alkyl;
$R^1$ is selected from the group consisting of hydrogen, haloalkyl, and $C_{3-6}$ cycloalkyl;
$R^2$ is selected from the group consisting of hydrogen, haloalkyl, and $C_{3-6}$ cycloalkyl;
or $R^1$ and $R^2$, together with the carbon atom to which they are attached, form $C_{3-6}$ cycloalkyl;
m is 0 or 1.

In some embodiments, the antibody-drug conjugate represented by general formula (I) (Pc-L-Y-D) or the pharmaceutically acceptable salt thereof according to any one of the foregoing is provided, wherein Y is selected from the group consisting of:

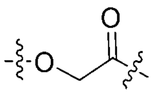, 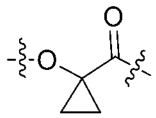, 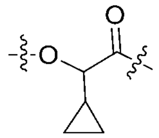, 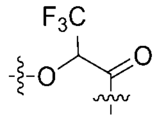, 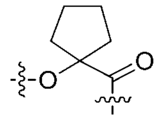, 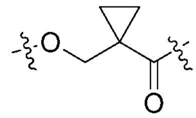,

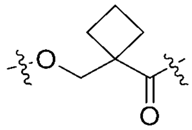, and 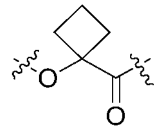,

wherein the O-end of Y is linked to the linker unit L.

In some embodiments, the antibody-drug conjugate represented by general formula (I) (Pc-L-Y-D) or the pharmaceutically acceptable salt thereof according to any one of the foregoing is provided, wherein the linker unit -L- is $-L^1-L^2-L^3-L^4-$;

$L^1$ is selected from the group consisting of -(succinimid-3-yl-*N*)-W-C(O)-, $-CH_2-C(O)-NR^3-W-C(O)-$, and -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)pCH_2CH_2C(O)-$, - $NR^4(CH_2CH_2O)pCH_2C(O)-$, $-S(CH_2)pC(O)-$, and a chemical bond, wherein p is an integer from 1 to 20;

$L^3$ is a peptide residue consisting of 2 to 7 amino acid residues, wherein the amino acid residues are selected from the group consisting of amino acid residues formed from amino acids from phenylalanine, glycine, valine, lysine, citrulline, serine, glutamic acid, and aspartic acid, and are optionally further substituted with one or more substituents selected from the group consisting of halogen, hydroxy, cyano, amino, alkyl, chloroalkyl, deuterated alkyl, alkoxy, and cycloalkyl;

$L^4$ is selected from the group consisting of $-NR^5(CR^6R^7)_t-$, -C(O)NR $^5$, $-C(O)NR^5(CH_2)_t-$, and a chemical bond, wherein t is an integer from 1 to 6;

$R^3$, $R^4$, and $R^5$ are identical or different and are each independently selected from the group consisting of hydrogen, alkyl, haloalkyl, deuterated alkyl, and hydroxyalkyl;

$R^6$ and $R^7$ are identical or different and are each independently selected from the group consisting of hydrogen, halogen, alkyl, haloalkyl, deuterated alkyl, and hydroxyalkyl.

In some embodiments, the antibody-drug conjugate represented by general formula (I) (Pc-L-Y-D) or the pharmaceutically acceptable salt thereof according to any one of the foregoing is provided, wherein the linker unit -L- is $-L^1-L^2-L^3-L^4-$;

$L^1$ is selected from the group consisting of -(succinimid-3-yl-*N*)-W-C(O)-, $-CH_2-C(O)-NR^3-W-C(O)-$, and -C(O)-W-C(O)-, wherein W is selected from the group consisting of $C_{1-8}$ alkyl, $C_{1-8}$ alkyl-cycloalkyl, and linear heteroalkyl of 1 to 8 chain atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)pCH_2CH_2C(O)-$, - $NR^4(CH_2CH_2O)pCH_2C(O)-$, $-S(CH_2)pC(O)-$, and a chemical bond, wherein p is an integer from 1 to 20;

$L^3$ is a peptide residue consisting of 2 to 7 amino acid residues, wherein the amino acid residues are selected from the group consisting of amino acid residues formed from amino acids from phenylalanine (F), glycine (G), valine (V), lysine (K), citrulline, serine (S), glutamic acid (Q), and aspartic acid (D), and are optionally further substituted with one or more substituents selected from the group consisting of halogen, hydroxy, cyano, amino, alkyl, chloroalkyl, deuterated alkyl, alkoxy, and cycloalkyl;

$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6, and non-limiting examples are 1, 2, 3, 4, 5, and 6;

$R^3$, $R^4$, and $R^5$ are identical or different and are each independently selected from the group consisting of hydrogen, alkyl, haloalkyl, deuterated alkyl, and hydroxyalkyl;

$R^6$ and $R^7$ are identical or different and are each independently selected from the group consisting of hydrogen, halogen, alkyl, haloalkyl, deuterated alkyl, and hydroxyalkyl.

In some embodiments, the antibody-drug conjugate represented by general formula (I) (Pc-L-Y-D) or the pharmaceutically acceptable salt thereof according to any one of the foregoing is provided, wherein the linker unit -L- is $-L^1-L^2-L^3-L^4-$;

$L^1$ is

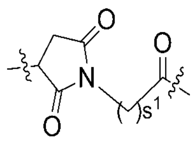

,

wherein $s^1$ is an integer from 2 to 8, and non-limiting examples are 2, 3, 4, 5, 6, 7, and 8;
$L^2$ is a chemical bond;
$L^3$ is a tetrapeptide residue; for example, $L^3$ is a tetrapeptide residue of GGFG;
$L^4$ is $-NR^5(CR^6R^7)_t-$, wherein $R^5$, $R^6$, and $R^7$ are identical or different and are each independently hydrogen or alkyl, and t is 1 or 2;
wherein the $L^1$ end is linked to Pc, and the $L^4$ end is linked to Y.

In some embodiments, the camptothecin drug is, e.g., exatecan.

In some embodiments, the antibody-drug conjugate represented by general formula (I) or the pharmaceutically acceptable salt thereof according to any one of the foregoing is an antibody-drug conjugate represented by general formula (II) or a pharmaceutically acceptable salt thereof:

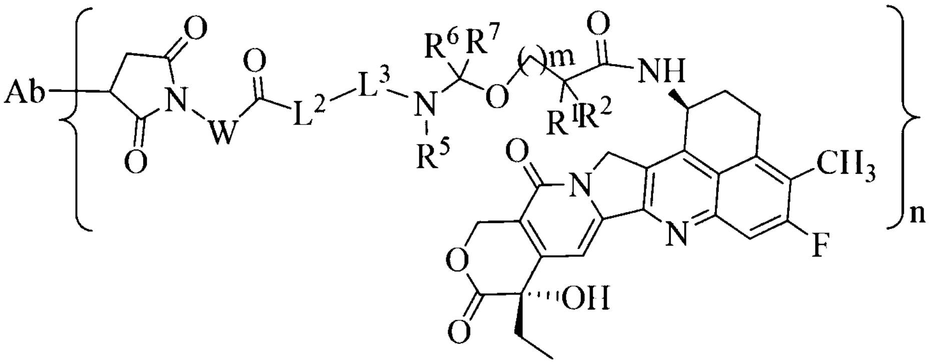

formula (II)

wherein:

W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$, and a chemical bond, and $p^1$ is an integer from 1 to 20;
$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;
$R^1$ is selected from the group consisting of hydrogen, halogen, cycloalkylalkyl, deuterated alkyl, cycloalkyl, heterocyclyl, aryl, and heteroaryl;
$R^2$ is selected from the group consisting of hydrogen, halogen, haloalkyl, deuterated alkyl, cycloalkyl, heterocyclyl, aryl, and heteroaryl;
or $R^1$ and $R^2$, together with the carbon atom to which they are attached, form cycloalkyl or heterocyclyl;
$R^4$ and $R^5$ are identical or different and are each independently selected from the group consisting of hydrogen, alkyl,

haloalkyl, deuterated alkyl, and hydroxyalkyl;
$R^6$ and $R^7$ are identical or different and are each independently selected from the group consisting of hydrogen, halogen, alkyl, haloalkyl, deuterated alkyl, and hydroxyalkyl; m is an integer from 0 to 4;
n is 1 to 10, and n is an integer or decimal;
Ab is as defined in general formula (I).

In some embodiments, the antibody-drug conjugate represented by formula (II) or the pharmaceutically acceptable salt thereof according to any one of the foregoing is provided, wherein n is 1 to 10, e.g., 1 to 8, 2 to 8, 2 to 7, 2 to 4, 3 to 8, 3 to 7, 3 to 6, 4 to 7, or 4 to 6, and n is a decimal or integer. In some embodiments, n is 1 to 8, and n is a decimal or integer. In some embodiments, n is 3 to 7, and n is a decimal or integer. In some embodiments, n is 4 to 6, and n is a decimal or integer. In some embodiments, n is an average value of about 1, about 2, about 3, about 4, about 5, about 6, about 7, about 8, about 9, or about 10. In some embodiments, n is an average value of about 4.1, about 4.2, about 4.3, about 4.4, about 4.5, about 4.6, 4.7, about 4.8, about 4.9, about 5, about 5.1, about 5.2, about 5.3, about 5.4, about 5.5, about 5.6, about 5.7, about 5.8, about 5.9, or about 6.

In some embodiments, the antibody-drug conjugate represented by formula (II) or the pharmaceutically acceptable salt thereof according to any one of the foregoing is provided, wherein:

the -L-Y- is represented by the following structure:

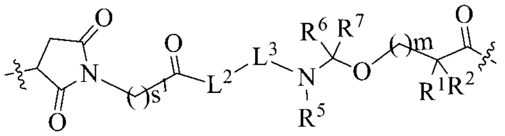

(-L-Y-) ;

$s^1$ is an integer from 2 to 8;
$L^2$, $L^3$, $R^1$, $R^2$, $R^5$, $R^6$, $R^7$, and m are as defined in the aforementioned general formula (II). In some embodiments, the antibody-drug conjugate represented by general formula (I) or the pharmaceutically acceptable salt thereof according to any one of the foregoing is an antibody-drug conjugate represented by general formula (III) ($Ab-L_b-Y-D$) or a pharmaceutically acceptable salt thereof:

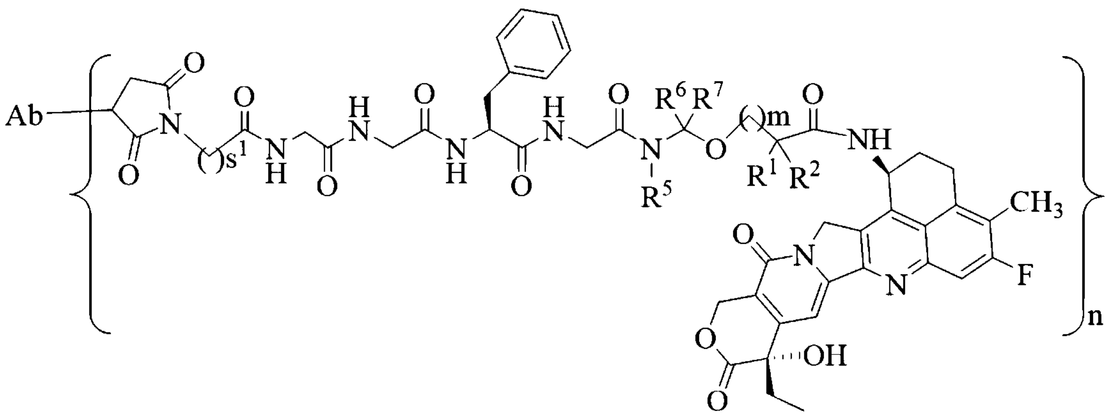

formula (III),

wherein:

$s^1$ is an integer from 2 to 8;
Pc, $R^1$, $R^2$, $R^5$, $R^6$, $R^7$, m, and n are as defined in general formula (II).

In some embodiments, the antibody-drug conjugate represented by general formula (I) or the pharmaceutically acceptable salt thereof according to any one of the foregoing is provided, wherein -L- is:

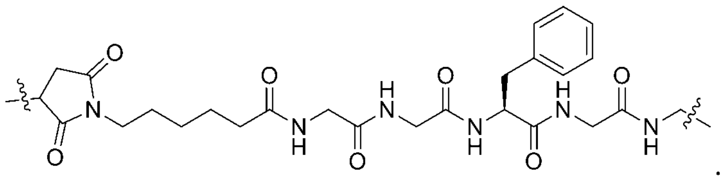

.

In some embodiments, the antibody-drug conjugate represented by general formula (I) or the pharmaceutically acceptable salt thereof according to any one of the foregoing is provided, wherein -L-Y- is selected from the group consisting of:

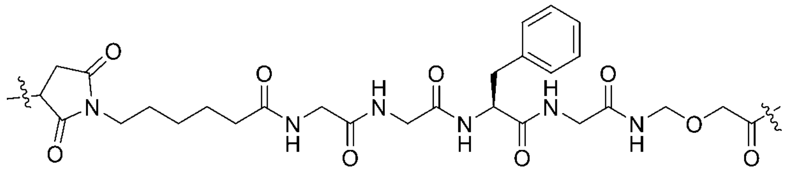

and

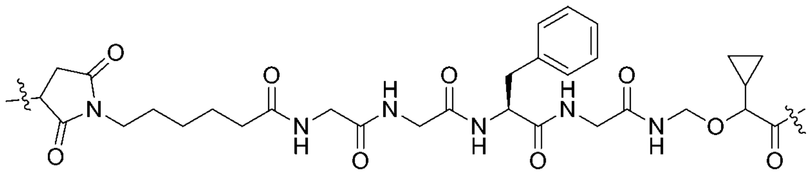

.

In some embodiments, -L-Y- is selected from the group consisting of:

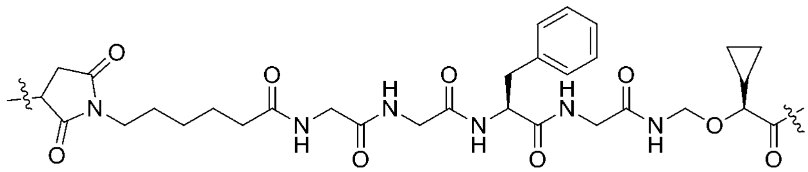

and

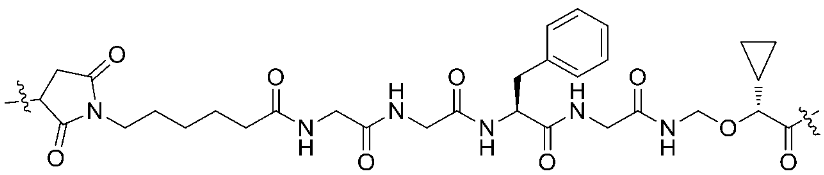

.

In some embodiments, -L-Y- is:

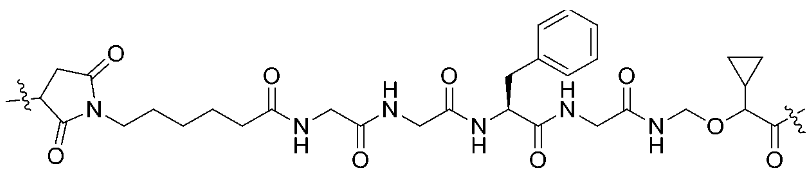

.

In some embodiments, -L-Y- is:

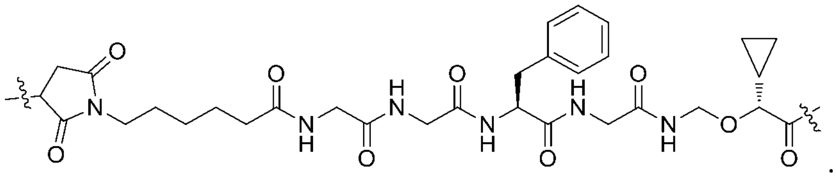

In some embodiments, the antibody-drug conjugate represented by general formula (I) or the pharmaceutically acceptable salt thereof according to any one of the foregoing is provided, wherein the antibody-drug conjugate is selected from the group consisting of:

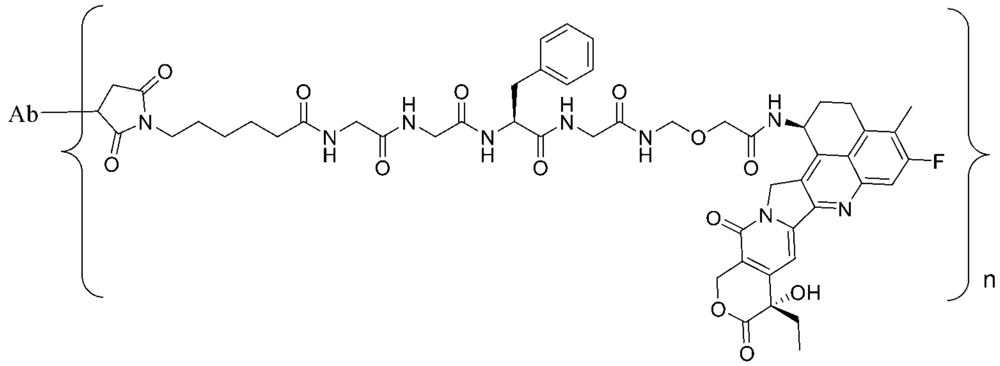

and

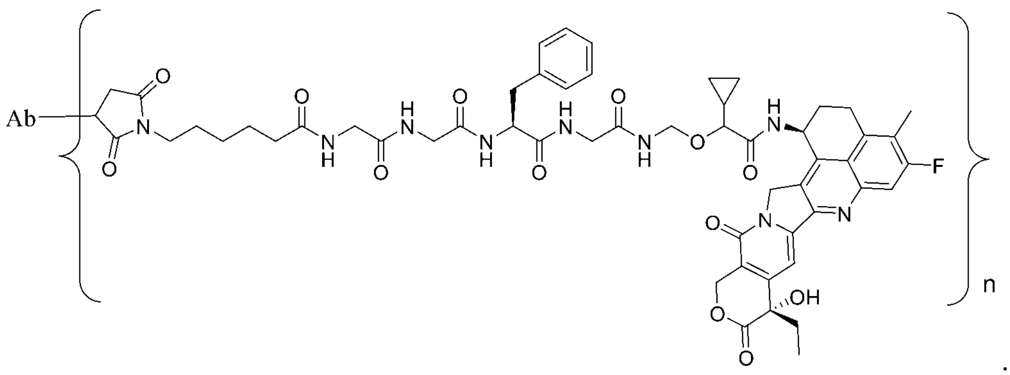

In some embodiments, the antibody-drug conjugate represented by general formula (I) or the pharmaceutically acceptable salt thereof according to any one of the foregoing is provided, wherein the antibody-drug conjugate is:

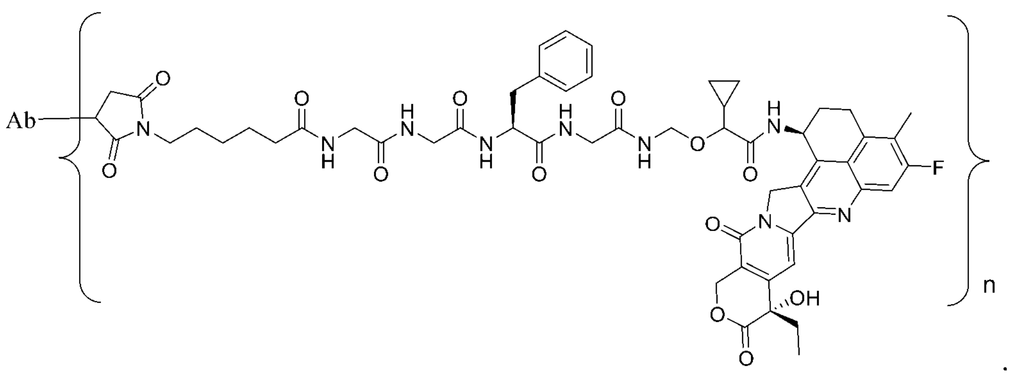

In some embodiments, the antibody-drug conjugate represented by general formula (I) or the pharmaceutically acceptable salt thereof according to any one of the foregoing is provided, wherein the antibody-drug conjugate is selected from the group consisting of:

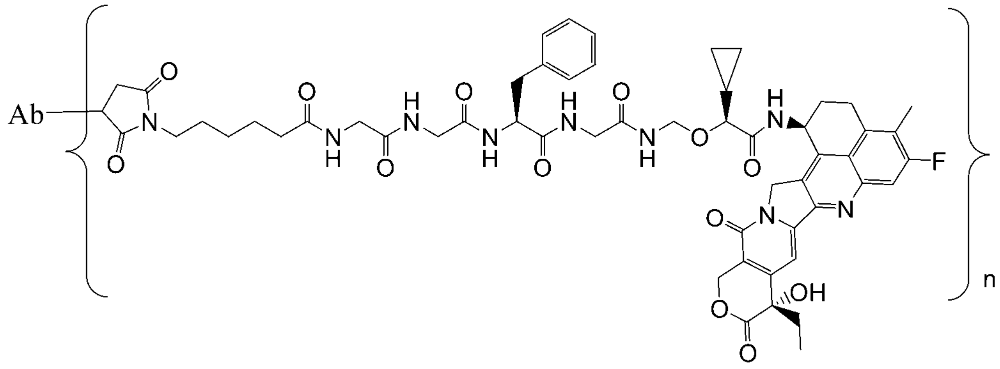

and

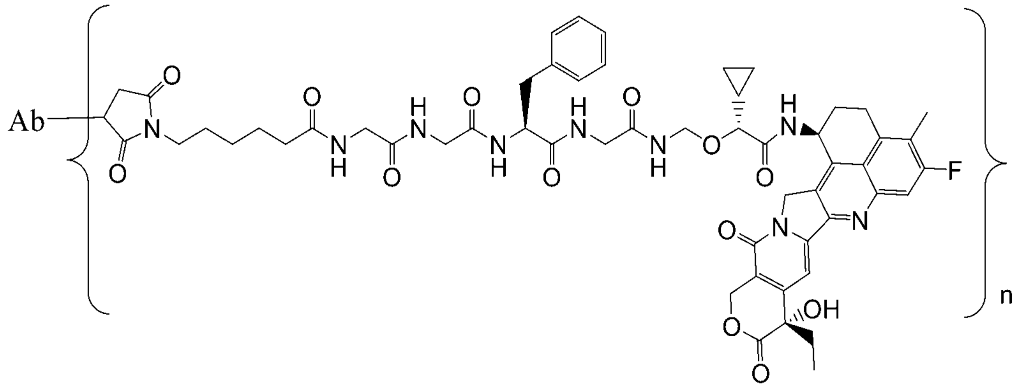

,

wherein Ab and n are as defined in general formula (I).

In some embodiments, the antibody-drug conjugate represented by general formula (I) or the pharmaceutically acceptable salt thereof according to any one of the foregoing is provided, wherein the antibody-drug conjugate is:

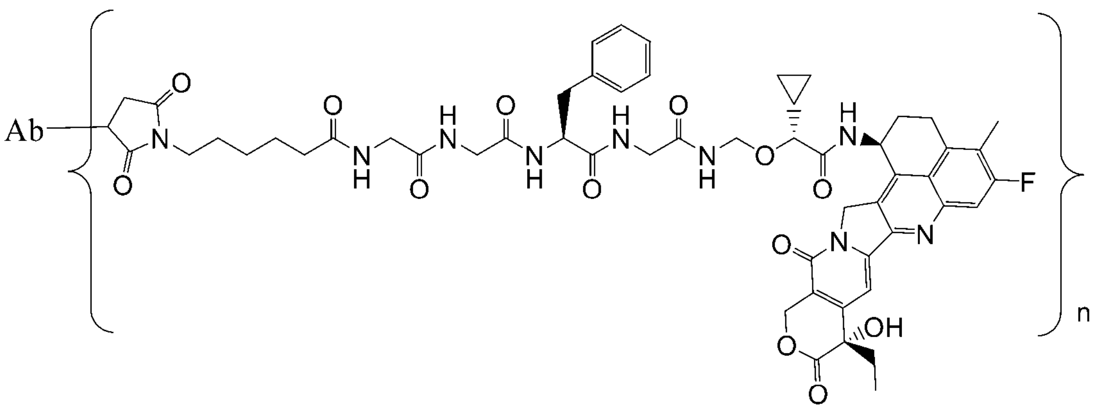

,

wherein:

n is 1 to 8, and n is a decimal or integer, e.g., an integer or decimal from 3 to 7, or, e.g., an integer or decimal from 4 to 6; Ab is an anti-c-Met antibody comprising a heavy chain set forth in SEQ ID NO: 18 and a light chain set forth in SEQ ID NO: 19.

Preparation Method

In some embodiments, the present disclosure provides a preparation method for an antibody-drug conjugate, comprising: subjecting any one of the aforementioned anti-c-Met antibodies to a coupling reaction with a drug to give the antibody-drug conjugate. Further, the method comprises: purifying the antibody-drug conjugate.

In some embodiments, a method for preparing the antibody-drug conjugate represented by general formula (I) is provided, comprising the following step:

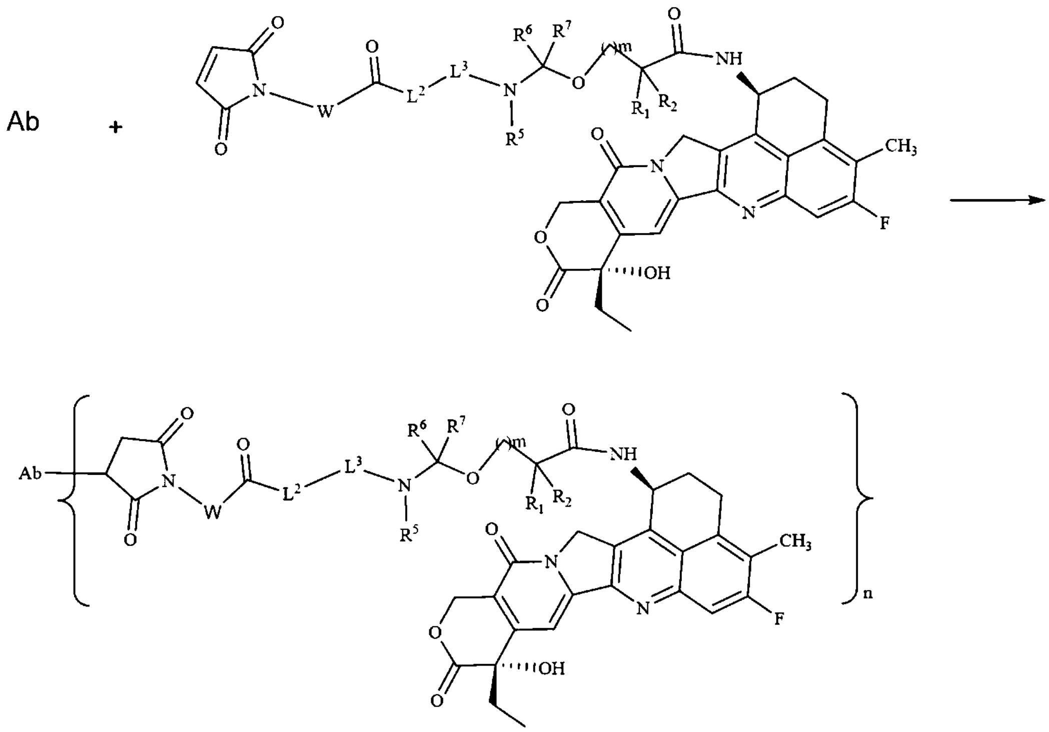

subjecting reduced Ab to a coupling reaction with general formula (La-Y-D) to give a compound represented by general formula (II),

wherein Ab is an anti-c-Met antibody of the present disclosure; W, $L^2$, $L^3$, $R^1$, $R^2$, $R^5$ to $R^7$, m, and n are as defined in formula (I).

In some embodiments, the reductant is TCEP.

In some embodiments, the reductant reduces disulfide bonds on the antibody.

Pharmaceutical Composition

The present disclosure provides a pharmaceutical composition, comprising the antibody-drug conjugate or the pharmaceutically acceptable salt thereof according to any one of the foregoing, and one or more pharmaceutically acceptable excipients, diluents, or carriers.

Pharmaceutical Use and Treatment Method

The present disclosure provides use of the antibody-drug conjugate or the pharmaceutically acceptable salt thereof according to any one of the foregoing or the pharmaceutical composition comprising same as a medicament.

The present disclosure provides use of the antibody-drug conjugate or the pharmaceutically acceptable salt thereof according to any one of the foregoing or the pharmaceutical composition comprising same in the manufacture of a medicament for treating a c-Met-mediated disease or disorder. In some embodiments, use of the antibody-drug conjugate or the pharmaceutically acceptable salt thereof according to any one of the foregoing for treating a cancer is provided. In some embodiments, the cancer is a cancer expressing c-Met. In some embodiments, the cancer is a cancer lowly, moderately, and/or highly expressing c-Met, e.g., a cancer moderately and/or highly expressing c-Met. In some embodiments, the cancer is gastric cancer, lung cancer, intestinal cancer, and/or liver cancer.

The antibody-drug conjugate according to any one of the foregoing provided by the present disclosure has tumor targeting, tumor cytotoxicity, and bystander killing, and can be used to inhibit the activity of tumor cells *in vitro* or in a subject *in vivo,* to kill tumor cells, and to treat c-Met-mediated related diseases.

In some specific embodiments, the present disclosure provides a method for treating a subject having a cancer or tumor or at risk of having a cancer, comprising: administering to the subject a therapeutically effective amount of an

antibody-drug conjugate or a pharmaceutical composition. In some embodiments, the cancer is a cancer expressing c-Met. In some embodiments, the cancer is gastric cancer, lung cancer, intestinal cancer, and/or liver cancer.

In some embodiments, the anti-c-Met antibody-drug conjugate or pharmaceutical composition of the present disclosure may be administered by any suitable method known in the art, including (but not limited to) parenteral administration (e.g., intradermal, intramuscular, intraperitoneal, intravenous, and subcutaneous) and intratumoral administration. The anti-c-Met antibody-drug conjugate or pharmaceutical composition may be administered by any suitable route, for example, by infusion or bolus injection. Administration may be systemic or local.

In some embodiments, the administration regimen may be adjusted to provide the best target response (e.g., a therapeutic or prophylactic response). For example, a single dose may be administered, several doses may be administered over a period of time, or the dose may be proportionally reduced or increased depending on the exigencies of the therapeutic situation.

## Definitions

In order to facilitate the understanding of the present disclosure, certain technical and scientific terms are specifically defined below. Unless otherwise specifically defined in the present disclosure, all other technical and scientific terms used in the present disclosure have the meanings generally understood by those of ordinary skill in the art to which the present disclosure pertains.

Unless the context clearly requires otherwise, throughout the description and the claims, the words "comprise", "have", "include", etc., are to be understood in an inclusive sense as opposed to an exclusive or exhaustive sense, that is to say, in the sense of "including but not limited to".

The three-letter and single-letter codes for amino acids used in the present disclosure are as described in J. biol. chem., 243, p3558 (1968).

The term "c-Met" or "c-Met polypeptide" or "c-Met receptor" refers to a receptor tyrosine kinase that binds to cell growth factor (HGF). In the present disclosure, unless otherwise specified, such as murine c-Met (m-c-Met) or monkey c-Met (cyno-c-Met), the term refers to human c-Met (h-c-Met). The human, murine, and cynomolgus monkey c-Met used in the present disclosure are all encoded by nucleotide sequences or polypeptide sequences provided by GenBank, for example, a human polypeptide encoded by the nucleotide sequence provided in GenBank Accession No. NM_000245, or a human protein encoded by the polypeptide sequence provided in GenBank Accession No. NP_000236 or its extracellular domain. The original single-chain precursor protein is post-translationally cleaved to produce $\alpha$ and $\beta$ subunits, which are linked by disulfide bonds to form a mature receptor. The receptor tyrosine kinase c-Met is involved in cell processes including, for example, the processes of migration, invasion, and morphogenesis of tissue regeneration that accompany embryogenesis.

The term "c-Met-related disorder or condition" refers to a disease, disorder, or condition that results from undesired expression or lack of expression, undesired regulation or lack of regulation, or undesired activity or lack of activity, of c-Met, or that can be modulated, treated, or cured by modulating c-Met expression or activity. For example, activation of the HGF/c-Met pathway can be expected in a large proportion of cancer patients, or in patients whose disease is really driven by alterations related to the c-Met pathway. For example, up-regulation is due to different mechanisms like overexpression of HGF and/or c-Met, or constitutive activation by c-Met mutation. c-Met-related disorders or conditions include, but are not limited to, e.g., proliferative diseases. The proliferative diseases include, but are not limited to, cancers including, e.g., gastric cancer, lung cancer, intestinal cancer, and/or liver cancer.

"Antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies and antibody fragments (or antigen-binding fragments, or antigen-binding moieties), so long as they exhibit the desired antigen-binding activity. An antibody may refer to an immunoglobulin, which is a four-peptide-chain structure formed by linking two heavy chains and two light chains by interchain disulfide bonds. The heavy chain constant regions of immunoglobulins differ in amino acid composition and arrangement; therefore, they also differ in antigenicity. Accordingly, immunoglobulins can be divided into five classes, or isotypes of immunoglobulins, namely IgM, IgD, IgG, IgA, and IgE, and their corresponding heavy chains are $\mu$ chains, $\delta$ chains, $\gamma$ chains, $\alpha$ chains, and $\varepsilon$ chains, respectively. Igs of the same class can be divided into different subclasses based on the amino acid composition of their hinge regions and the number and positions of heavy chain disulfide bonds; for example, IgGs can be divided into IgG1, IgG2, IgG3, and IgG4. Light chains are divided into $\kappa$ or $\lambda$ chains based on their constant regions.

Each of the five Ig classes may have a $\kappa$ chain or $\lambda$ chain.

In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity determining regions

(CDRs). Each light chain variable region (VL) or heavy chain variable region (VH) consists of 3 CDRs and 4 FRs arranged from the amino-terminus to the carboxyl-terminus in the following order: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3. In the present disclosure, the antibody light chain may further comprise a light chain constant region. Illustratively, the light chain constant region comprises a human or murine $\kappa$ or $\lambda$ chain or a variant thereof.

In the present disclosure, the antibody heavy chain may further comprise a heavy chain constant region. Illustratively, the heavy chain constant region comprises human or murine IgG1, IgG2, IgG3, IgG4, or a variant thereof.

The term "murine antibody" in the present disclosure refers to an anti-human c-Met monoclonal antibody prepared using mice according to the knowledge and skills in the art. During the preparation, the c-Met antigen is injected into a test subject, and then hybridomas expressing antibodies with the desired sequences or functional properties are isolated. In an embodiment of the present disclosure, the murine c-Met antibody may further comprise the light chain constant region of a murine $\kappa$ or $\lambda$ chain or a variant thereof, or further comprise the heavy chain constant region of murine IgG1, IgG2, IgG3, or IgG4 or a variant thereof.

The term "chimeric antibody" refers to an antibody obtained by fusing a variable region of a murine antibody to a constant region of a human antibody, which can reduce an immune response induced by the murine antibody. To construct a chimeric antibody, a hybridoma secreting a murine specific monoclonal antibody should be constructed first, and the variable region gene is cloned from mouse hybridoma cells and then cloned into the constant region gene of a human antibody for recombinant expression.

The term "humanized antibody", also known as CDR-grafted antibody humanization, refers to an antibody produced by grafting murine CDR sequences into a human antibody variable region framework, i.e., a different type of human germline antibody framework sequence. The strong antibody response induced by the chimeric antibody due to the large amount of mouse protein components it carries can be overcome. Such framework sequences can be obtained from public DNA databases or published references that include germline antibody gene sequences. For example, the germline DNA sequences of genes of human heavy and light chain variable regions can be found in the "VBase" human germline sequence database, as well as in Kabat, E. A. et al., 1991 Sequences of Proteins of Immunological Interest, 5th edition. Human antibody variable region frameworks are designed and selected, wherein the light chain FR sequences in the antibody light chain variable region are selected from the group consisting of human germline light chain sequences, e.g., the human germline light chain IGKV085 or IGKV 4-1*01, and comprise the FR1, FR2, FR3, and FR4 of the human germline light chain IGKV085 or IGKV 4-1*01, and the heavy chain FR sequences in the antibody heavy chain variable region are derived from a human germline heavy chain sequence, e.g., the human germline heavy chain IGHV 3-33*01, and comprise the FR1, FR2, FR3, and FR4 of the human germline heavy chain IGHV 3-33*01. To avoid a decrease in activity caused by a decrease in immunogenicity, the variable regions of a human antibody can be subjected to the minimum number of back mutations to maintain activity.

The term "antigen-binding site" may be a discontinuous three-dimensional spatial site on an antigen that is recognized by the antibody or the antigen-binding fragment of the present disclosure.

The term "epitope" refers to a site on an antigen to which an immunoglobulin or an antibody binds. An epitope can be formed from contiguous amino acids, or non-contiguous amino acids (non-contiguous amino acids are brought into spatial proximity by tertiary folding of the protein). An epitope formed from contiguous amino acids is generally retained after exposure to a denaturing solvent, while an epitope formed by tertiary folding is generally lost after treatment with a denaturing solvent. An epitope generally exists in a unique spatial conformation and comprises at least 3-15 amino acids. Methods for determining an epitope are well known in the art and include immunoblotting, immunoprecipitation assay and analysis, etc. Methods for determining the spatial conformation of an epitope include techniques in the art and techniques described herein, such as X-ray crystallography and two-dimensional nuclear magnetic resonance.

"Specific binding" or "selective binding" refers to the binding of an antibody to an epitope on a predetermined antigen. Generally, an antibody binds to a predetermined antigen or epitope thereof with an equilibrium dissociation constant ($K_D$) of about less than $10^{-7}$ M or even less and with an affinity that is at least twice as high as the affinity with which it binds to the predetermined antigen or the epitope thereof (or non-specific antigens apart from closely related antigens, e.g., BSA), as determined by surface plasmon resonance (SPR) technology in an instrument with human c-Met or an epitope thereof as an analyte and the antibody as an antibody.

"Affinity" refers to the overall strength of a non-covalent interaction between a single binding site of a molecule (e.g., an antibody) and its binding antibody (e.g., an antigen).

Unless otherwise indicated, as used herein, "binding affinity" refers to an internal binding affinity that reflects a 1:1 interaction between the members of a binding pair (e.g., an antibody and an antigen). Generally, the affinity of molecule X for its antibody Y can be expressed in terms of the equilibrium dissociation constant (KD). Affinity can be measured by conventional methods known in the art, including those described herein. The term "kassoc" or "ka" refers to the association rate of a particular antibody-antigen interaction, while the term "kdis" or "kd" as used herein is intended to refer to the dissociation rate of a particular antibody-antigen interaction. As used herein, the term "KD" refers to the equilibrium dissociation constant, which is obtained from the ratio of kd to ka (i.e., kd/ka) and expressed as molar

concentration (M). The KD values of antibodies can be determined by methods known in the art; for example, methods for determining antibody KD include measuring surface plasmon resonance using a biosensing system, such as a system, or measuring affinity in a solution by solution equilibrium titration (SET). "Cross-reactivity" refers to the ability of an antibody (or a fragment thereof) of the present disclosure to bind to c-Met from different species. For example, an antibody of the present disclosure that binds to human c-Met may also bind to c-Met from another species. Cross-reactivity is measured by testing the specific reactivity with a purified antigen in a binding assay (e.g., SPR or ELISA) or through binding or functional interactions with cells physiologically expressing c-Met. Methods for determining cross-reactivity include standard binding assays as described herein, for example, surface plasmon resonance analysis or flow cytometry.

"Homology" or "identity" refers to the sequence similarity between two polynucleotide sequences or two polypeptides. When positions in two sequences being compared are occupied by identical bases or amino acid monomer subunits, for example, if a position in each of two DNA molecules is occupied by adenine, the molecules are homologous at that position. The percent homology between two sequences is a function of the number of matching or homologous positions shared by the two sequences divided by the number of compared positions × 100%. For example, if 6 out of 10 positions are matched or homologous when two sequences are optimally aligned, the two sequences are 60% homologous. In general, when two sequences are aligned, a comparison is performed to obtain the maximum percent homology. In order to determine the percent identity of amino acid sequences, an alignment can be achieved in a variety of ways that fall within the skill in the art, for example, using computer software such as BLAST, BLAST-2, ALIGN, ALIGN-2, or Megalign (DNASTAR) software. Those skilled in the art can determine parameters suitable for measuring an alignment, including any algorithm required to achieve the maximum alignment of the full length of the sequences being compared, such as BLAST.

The term "internalization" refers to the transport of a moiety from the outside of a cell to the inside. The internalized moiety can be located in an intracellular compartment. An "internalized" antigen or antibody refers to an antigen or antibody that is capable of being transported from the outside of a target cell to the inside. Those skilled in the art will readily appreciate that the process of cellular internalization generally refers to the movement of a cell-surface molecule across the plasma membrane from the cell surface to the inside of the cell. After internalization, endosomes can be trafficked to lysosomes for degradation or recycled to the cell surface. The cellular internalization rate of a given cell-surface molecule provides a measurement of the kinetics of the movement of the molecule from the cell surface to the inside of the cell through the plasma membrane. The internalization activity or rates of antigens and antibodies can be monitored and/or measured by a number of techniques known in the art, including acid dissociation (Li N. et al., Methods Mol. Biol., 457:305-17, 2008) and toxin-killing assays (Pahara J. et al., Exp Cell Res., 316:2237-50, 2010; and Mazor et al., J. Immunol. Methods, 321:41-59, 2007). Many antibody labeling techniques, dyes, and kits for antibody labeling that can be used to quantify and monitor internalization are commercially available (e.g., commercially available pHrodo iFL antibody labeling methods, reagents, and kits from Thermo Fisher Scientific).

For example, an antibody-drug conjugate (ADC) binds to the tumor cell surface antigen through the antibody in the ADC and is then endocytosed, entering an endosome. Then the ADC is trafficked from the endosome to a lysosome, and the biologically active molecule (e.g., a toxin or payload) is dissociated from the ADC by the action of the hydrolase in the lysosome, enters the cytoplasm from the lysosome, and kills the tumor cell. The biologically active molecule that escapes from the killed tumor cell can further kill neighboring tumor cells that do not express the antigen or lowly express the antigen (the so-called bystander effect).

"Mutant" or "variant" refers to a polypeptide comprising at least one amino acid mutation (such as a substitution, deletion, or insertion) as compared to the "parent" amino acid sequence, so long as the variant is still capable of binding to the antigen or fragments thereof. Amino acid mutations (also referred to as amino acid modifications) include, for example, deletions and/or insertions and/or substitutions of residues in the amino acid sequence of a binding molecule (e.g., an antibody). Any combination of deletions, insertions, and substitutions may be introduced into a "parent" amino acid sequence to arrive at a final variant. The amino acid mutation also includes changes in post-translational processes of the binding molecule, such as changes in the number or position of glycosylation sites. For example, depending on the length of the CDRs and FRs themselves, 1, 2, 3, 4, 5, or 6 amino acids may be inserted into or deleted from each CDR, and 1, 2, 3, 4, 5, 6, 7, 8, 9, 10, 11, 12, 13, 14, 15, 16, 17, 18, 19, 20, or 25 amino acids may be inserted into or deleted from each FR. Moreover, amino acid substitutions may be introduced into the CDRs, VHs, or FRs of the heavy and/or light chains. Examples are conservative substitutions.

"Conservative substitution" refers to the replacement with another amino acid residue having similar properties to the original amino acid residue. For example, lysine, arginine, and histidine have similar properties in that they have basic side chains, and aspartic acid and glutamic acid have similar properties in that they have acidic side chains. Additionally, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is substituted, it will

not exhibit a particular change in properties.

The term "camptothecin drug" refers to camptothecin, which is cytotoxic, and derivatives thereof, which are selected from the group consisting of, without limitation, 10-hydroxycamptothecin, 7-ethyl-10-hydroxycamptothecin, topotecan, exatecan, irinotecan, or 9-nitro-10-hydroxycamptothecin, and derivatives or pharmaceutically acceptable salts thereof.

The term "antibody-drug conjugate" (ADC) means that an antibody is linked to a drug by a linker (or a linker unit). In some embodiments, the "antibody-drug conjugate" in the present disclosure means that an anti-C-met antibody is linked to a toxic drug by a linker unit.

The term "drug" refers to a cytotoxic drug. The drug is represented by D. It is a chemical molecule that can strongly disrupt the normal growth of tumor cells. A cytotoxic drug can kill tumor cells in principle at a sufficiently high concentration; however, due to the lack of specificity, it can cause apoptosis of normal cells while killing tumor cells, resulting in serious side effects. The term includes toxins, such as small-molecule toxins or enzymatically active toxins derived from bacteria, fungi, plants, or animals, radioisotopes (e.g., $At^{211}$, $I^{131}$, $I^{125}$, $Y^{90}$, $Re^{186}$, $Re^{188}$, $Sm^{153}$ , $Bi^{212}$, $P^{32}$, and radioactive isotopes of Lu), toxic drugs, chemotherapeutic drugs, antibiotics, and nucleolytic enzymes. Toxic drugs are preferred.

The term "linker unit" or "linker fragment" refers to a chemical structure fragment or bond that is linked to an antibody at one end and to a drug at the other end, and it may also be linked to a drug after being linked to another linker. In a non-limiting embodiment, it is represented by L and L1 to L4, wherein the L1 end is linked to the antibody, and the L4 end is linked to the structural unit Y and then linked to the drug (D).

The term "drug loading" refers to the average number of cytotoxic drugs loaded onto each ligand (anti-c-Met antibody) in an ADC, and it may also be expressed as the ratio of the number of drugs to the number of antibodies. The drug loading range may be 1-20, e.g., 1-10, cytotoxic drugs (D) linked to each antibody (Pc). In embodiments of the present disclosure, drug loading is represented by n, and is illustratively an average value of 1, 2, 3, 4, 5, 6, 7, 8, 9, 10, 11, 12, 13, 14, 15, 16, 17, 18, 19, 20, or a value between any two of these values. Thus, drug loading may be an integer or decimal. The average number of drugs per ADC molecule after a coupling reaction can be characterized by conventional methods such as UV/visible spectroscopy, mass spectrometry, an ELISA assay, a monoclonal antibody molecule size variant assay (CE-SDS), and HPLC.

The monoclonal antibody molecular size variant assay (CE-SDS) of the present disclosure may be used for quantitatively determining the purity of a recombinant monoclonal antibody product by adopting a capillary electrophoresis-sodium dodecyl sulfate (CE-SDS) ultraviolet assay based on the molecular weight under reduced and nonreduced conditions and according to a capillary electrophoresis method (Chinese Pharmacopoeia 0542, 2015 Edition).

Although the drug-to-antibody ratio has an exact value (e.g., n in formula (I)) for a specific conjugate molecule, it will be understood that when used to describe a sample containing many molecules, the value will often be a mean, which is attributed to a certain degree of non-uniformity typically associated with the conjugation step. The mean loading of an immunoconjugate sample is referred to herein as the drug-to-antibody ratio or "DAR". In some examples, the DAR is between about 1 and about 10, e.g., 1-8, and is typically about 1, 1.5, 2, 2.5, 3, 3.5, 4, 4.5, 5, 5.5, 6, 6.5, 7.0, 7.5, or 8.0. In some examples, at least 50% by weight of the sample is a compound having a mean DAR $\pm$ 2; in some specific examples, at least 50% by weight of the sample is a conjugate having a mean DAR $\pm$ 1. Examples include immunoconjugates in which the DAR is about 1.1, about 1.2, about 1.3, about 1.4, about 1.5, about 1.6, about 1.7, about 1.8, about 1.9, about 2.0, about 2.1, about 2.2, about 2.3, about 2.4, about 2.5, about 2.6, about 2.7, about 2.8, about 2.9, about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, about 5.0, about 5.1, about 5.2, about 5.4, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, or about 7.0. In some examples, a DAR of 'about x' means that the DAR measurement is within 20% of x.

The method of determining DAR is, for example, extrapolating a DAR value from LC-MS data of reduced and deglycosylated samples. LC/MS allows for quantification of the average number of payload (drug moiety) molecules linked to the antibody in an ADC. HPLC separates an antibody into light and heavy chains, and also separates the heavy (HC) and light (LC) chains according to the number of linker-payload groups in each chain. Mass spectrometry data enables identification of the types of components in a mixture, e.g., LC, LC+1, LC+2, HC, HC+1, and HC+2. From the mean loading of the LC and HC chains, the mean DAR of an ADC can be calculated. The DAR of a given immunoconjugate sample represents the average number of drug (payload) molecules linked to a tetrameric antibody containing two light chains and two heavy chains. An example is the DAR determination method described in WO2018142322.

The term "alkyl" refers to a saturated aliphatic hydrocarbon group that is a linear or branched group containing 1 to 20 carbon atoms, e.g., an alkyl group containing 1 to 12 carbon atoms, an alkyl group containing 1 to 10 carbon atoms, or an alkyl group containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. For example, non-limiting examples of lower alkyl groups containing 1 to 6 carbon atoms include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted, and when it is substituted, the substituent may be substituted at any available point of attachment. In non-limiting examples, the substituent is one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "heteroalkyl" refers to an alkyl group containing one or more heteroatoms selected from the group consisting of N, O, and S, wherein the alkyl is as defined above. The term "alkylene" refers to a saturated linear or branched aliphatic hydrocarbon group having 2 residues derived by removing two hydrogen atoms from the same carbon atom or two different carbon atoms of the parent alkane, and it is a linear or branched group containing 1 to 20 carbon atoms, e.g., an alkylene group containing 1 to 12 carbon atoms or 1 to 6 carbon atoms. Non-limiting examples of alkylene groups include, but are not limited to, methylene ($-CH_2-$), 1,1-ethylidene ($-CH(CH_3)-$), 1,2-ethylidene ($-CH_2CH_2$)-, 1,1-propylidene ($-CH(CH_2CH_3)-$), 1,2-propylidene ($-CH_2CH(CH_3)-$), 1,3-propylidene ($-CH_2CH_2CH_2-$), 1,4-butylidene ($-CH_2CH_2CH_2CH_2-$), 1,5-butylidene ($-CH_2CH_2CH_2CH_2CH_2-$), etc. Alkylene may be substituted or unsubstituted, and when it is substituted, the substituent may be substituted at any available point of attachment. In non-limiting examples, the substituent is substituted with one or more substituents independently and optionally selected from the group consisting of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "alkoxy" refers to -O-(alkyl) and -O-(unsubstituted cycloalkyl), wherein the alkyl or cycloalkyl is as defined above. Non-limiting examples of alkoxy groups include methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent may be one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "cycloalkyl" refers to a saturated or partially unsaturated monocyclic or polycyclic hydrocarbon substituent, and the cycloalkyl ring contains 3 to 20 carbon atoms, e.g., 3 to 12 carbon atoms, 3 to 10 carbon atoms, or 3 to 8 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl groups include spiro-ring, fused-ring, and bridged-ring cycloalkyl groups.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen, and $S(O)_m$ (where m is an integer from 0 to 2), but a ring moiety of -O-O-, -O-S-, or -S-S- is excluded, and the other ring atoms are carbon atoms. For example, it contains 3 to 12 ring atoms, of which 1 to 4 are heteroatoms; or it contains 3 to 10 ring atoms. Non-limiting examples of monocyclic heterocyclyl groups include pyrrolidinyl, piperidinyl, piperazinyl, morpholinyl, thiomorpholinyl, homopiperazinyl, etc. Polycyclic heterocyclyl groups include spiro-ring, fused-ring, and bridged-ring heterocyclyl groups.

The term "spiroheterocyclyl" refers to a 5- to 20-membered (e.g., 6- to 14-membered or 7- to 10-membered) polycyclic heterocyclyl group in which one atom (referred to as a spiro atom) is shared between monocyclic rings, wherein one or more ring atoms are heteroatoms selected from the group consisting of nitrogen, oxygen, and $S(O)_m$ (where m is an integer from 0 to 2), and the other ring atoms are carbon atoms. It may contain one or more double bonds, but no ring has a fully conjugated $\pi$-electron system. According to the number of spiro-atoms shared between rings, spiroheterocyclyl may be monospiroheterocyclyl, bispiroheterocyclyl, or polyspiroheterocyclyl, for example, 4-membered/4-membered, 4-membered/5-membered, 4-membered/6-membered, 5-membered/5-membered, or 5-membered/6-membered monospiroheterocyclyl. Non-limiting examples of spiroheterocyclyl groups include:

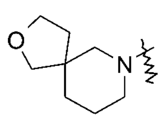 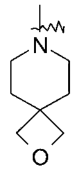 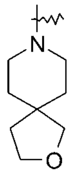 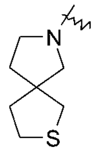 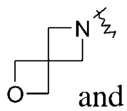 and 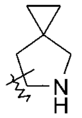 .

The term "fused heterocyclyl" refers to a 5- to 20-membered (e.g., 6- to 14-membered or 7- to 10-membered) polycyclic heterocyclyl group in which each ring in the system shares a pair of adjacent atoms with the other rings in the system, wherein one or more rings may contain one or more double bonds, but no ring has a fully conjugated π-electron system; one or more ring atoms are heteroatoms selected from the group consisting of nitrogen, oxygen, and $S(O)_m$ (where m is an integer from 0 to 2), and the other ring atoms are carbon atoms. According to the number of constituent rings, it may be bicyclic, tricyclic, tetracyclic, or polycyclic fused heterocyclyl, for example, 5-membered/5-membered or 5-membered/6-membered bicyclic fused heterocyclyl. Non-limiting examples of fused heterocyclyl groups include:

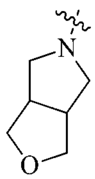 , 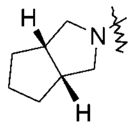 , 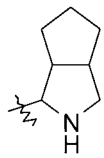 , 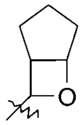 , 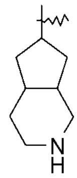 , 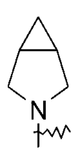 , 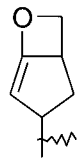 , 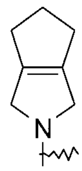 ,

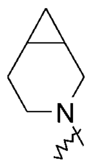 , 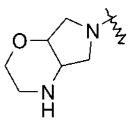 , 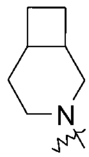 , 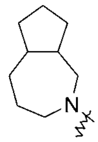 , 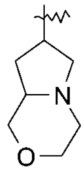 , 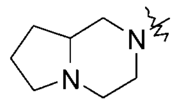 ,

and

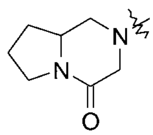 .

The term "bridged heterocyclyl" refers to a 5- to 14-membered (e.g., 6- to 14-membered or 7- to 10-membered) polycyclic heterocyclyl group in which any two rings share two atoms that are not directly linked, and it may contain one or more double bonds, but no ring has a fully conjugated π-electron system, wherein one or more ring atoms are heteroatoms selected from the group consisting of nitrogen, oxygen, and $S(O)_m$ (where m is an integer from 0 to 2), and the other ring atoms are carbon atoms. According to the number of constituent rings, it may be bicyclic, tricyclic, tetracyclic, or polycyclic bridged heterocyclyl. Non-limiting examples of bridged heterocyclyl groups include:

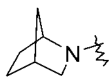 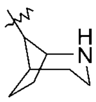 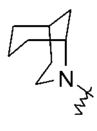 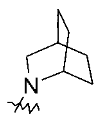

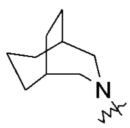 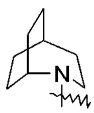 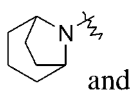 and 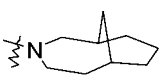 .

The heterocyclyl ring may be fused to an aryl, heteroaryl, or cycloalkyl ring, wherein the ring attached to the parent structure is heterocyclyl; its non-limiting examples include:

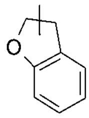, 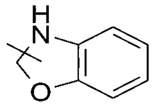, 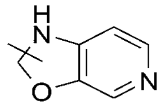, 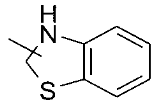,

etc.

Heterocyclyl may be optionally substituted or unsubstituted, and when it is substituted, the substituent may be one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "aryl" refers to a 6- to 14-membered, e.g., 6- to 10-membered, all-carbon monocyclic or fused polycyclic (i.e., rings sharing a pair of adjacent carbon atoms) group having a conjugated system, such as phenyl or naphthyl. The aryl ring may be fused to a heteroaryl, heterocyclyl, or cycloalkyl ring, wherein the ring attached to the parent structure is the aryl ring; its non-limiting examples include:

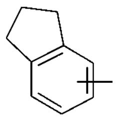, 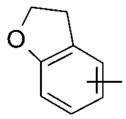, 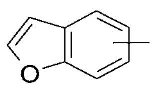, 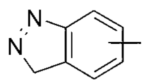, 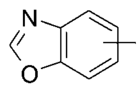, 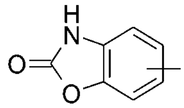,

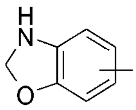, 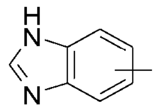, 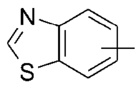, 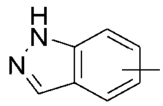, 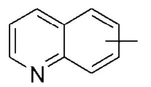, 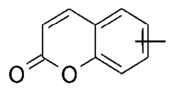,

and

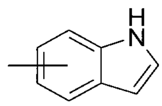.

Aryl may be substituted or unsubstituted, and when it is substituted, the substituent may be one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. In non-limiting examples, heteroaryl is 5- to 10-membered, or 5- or 6-membered, e.g., furanyl, thienyl, pyridyl, pyrrolyl, N-alkylpyrrolyl, pyrimidinyl, pyrazinyl, imidazolyl, and tetrazolyl. The heteroaryl ring may be fused to an aryl, heterocyclyl, or cycloalkyl ring, wherein the ring attached to the parent structure is the heteroaryl ring; its non-limiting examples include:

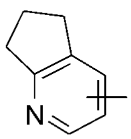, 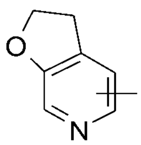, 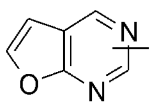, 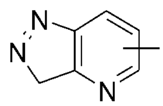, 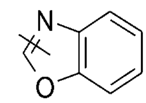, 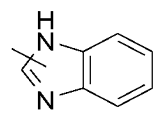,

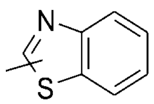, 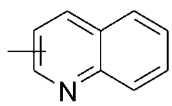, 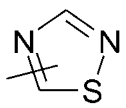, and 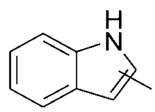.

Heteroaryl may be optionally substituted or unsubstituted, and when it is substituted, the substituent may be one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "amino protecting group" refers to an easily removable group for protecting an amino group from being

changed when a reaction is taking place elsewhere in the molecule. Non-limiting examples include 9-fluorenylmethoxycarbonyl, tert-butoxycarbonyl, acetyl, benzyl, allyl, p-methoxybenzyl, etc. These groups may be optionally substituted with 1-3 substituents selected from the group consisting of halogen, alkoxy, and nitro. For example, the amino protecting group is 9-fluorenylmethoxycarbonyl.

The term "cycloalkylalkyl" refers to an alkyl group substituted with one or more cycloalkyl groups, wherein the alkyl is as defined above, and the cycloalkyl is as defined above.

The term "haloalkyl" refers to an alkyl group substituted with one or more halogens, wherein the alkyl is as defined above.

The term "deuterated alkyl" refers to an alkyl group substituted with one or more deuterium atoms, wherein the alkyl is as defined above.

The term "hydroxy" refers to the -OH group.

The term "halogen" refers to fluorine, chlorine, bromine, or iodine.

The term "amino" refers to $-NH_2$.

The term "nitro" refers to $-NO_2$.

The term "acylamino" refers to -C(O)N(alkyl) or (cycloalkyl), wherein the alkyl and cycloalkyl are as defined above.

The term "carboxylate group" refers to -C(O)O(alkyl) or (cycloalkyl), wherein the alkyl and cycloalkyl are as defined above.

The present disclosure also includes various deuterated forms of the compound of formula (I). Each available hydrogen atom linked to a carbon atom may be independently replaced with a deuterium atom. Those skilled in the art are able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, etc.

"Optional" or "optionally" means that the event or circumstance subsequently described may, but does not necessarily, occur; this description includes an instance where the event or circumstance occurs or does not occur. For example, "a heterocyclyl group optionally substituted with alkyl" means that alkyl may, but does not necessarily, exist; this description includes an instance where the heterocyclyl group is substituted with alkyl and an instance where it is not.

"Substituted" means that one or more (up to 5 in non-limiting examples), e.g., 1 to 3, hydrogen atoms in the group are independently substituted with a corresponding number of substituents. It goes without saying that a substituent is only in its possible chemical positions, and those skilled in the art will be able to determine (experimentally or theoretically) possible or impossible substitutions without undue effort. For example, the binding of an amino or hydroxy group having free hydrogen to a carbon atom having an unsaturated (e.g., olefinic) bond may be unstable.

"Giving", "administering", and "treating", when applied to animals, humans, experimental subjects, cells, tissues, organs, or biological fluid, refer to contact of an exogenous drug, a therapeutic agent, a diagnostic agent, or a composition with the animals, humans, subjects, cells, tissues, organs, or biological fluid. "Giving", "administering", and "treating" can refer to, for example, therapeutic, pharmacokinetic, diagnostic, research, and experimental methods. The treatment of cells comprises contacting a reagent with cells and contacting the reagent with a fluid, wherein the fluid is in contact with the cells. "Giving", "administering", and "treating" also mean treating, e.g., a cell, by a reagent, diagnosis, a binding composition, or by another cell *in vitro* and *ex vivo.* "Treating", when applied to human, veterinary, or research subjects, refers to therapeutic treatment, preventive or prophylactic measures, and research and diagnostic applications. "Treatment" means administering a therapeutic agent, such as a composition comprising any one of the antibody-drug conjugates of the present disclosure, either internally or externally to a subject who has had, is suspected of having, or is predisposed to having one or more diseases or symptoms thereof on which the therapeutic agent is known to have a therapeutic effect. Generally, the therapeutic agent is administered in an amount effective to alleviate one or more disease symptoms in the subject or population being treated, whether by inducing regression of such symptoms or inhibiting the development of such symptoms to any clinically measurable degree. The amount of therapeutic agent effective to alleviate any specific disease symptom (also referred to as the "therapeutically effective amount") may vary depending on factors such as the disease state, age, and body weight of the subject, and the ability of the drug to produce a desired therapeutic effect in the subject. Whether a disease symptom has been palliated can be evaluated by any clinical testing methods commonly used by doctors or other health care professionals to evaluate the severity or progression of the symptom. Although embodiments of the present disclosure (e.g., treatment methods or products) may be ineffective in alleviating symptoms of a disease of interest in a certain subject, they shall palliate the symptoms of the disease of interest in a statistically significant number of subjects, as determined by any statistical test method known in the art, such as Student's t-test, Chi-square test, U-test by Mann and Whitney, Kruskal-Wallis test (H-test), Jonckheere-Terpstra test, and Wilcoxon test.

"Effective amount" comprises an amount sufficient to ameliorate or prevent a symptom of a medical disorder. An

effective amount also means an amount sufficient to allow or facilitate diagnosis. The effective amount for a particular subject or veterinary subject may vary depending on factors such as the disorder to be treated, the general health of the subject, the method, route, and dose of administration, and the severity of side effects. An effective amount may be the maximum dose or administration regimen to avoid significant side effects or toxic effects.

The term "drug carrier", when used for the drug of the present disclosure, refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects, and improve bioavailability. For example, polymeric surfactants that can be used as carriers can self-assemble due to their distinctive amphiphilic structures to form various forms of aggregates; non-limiting examples are, e.g., micelles, microemulsions, gels, liquid crystals, and vesicles. These aggregates have the ability to encapsulate drug molecules and have good permeability for membranes; therefore, they can be used as excellent drug carriers.

The term "excipient" is an addition, besides the main drug, to a pharmaceutical formulation. It may also be referred to as an auxiliary material. For example, the binders, fillers, disintegrants, lubricants in tablets, the matrix part in semisolid ointment and cream formulations, the preservatives, antioxidants, corrigents, fragrances, cosolvents, emulsifiers, solubilizers, osmotic pressure regulators, and colorants in liquid formulations, etc., can all be referred to as excipients.

The term "diluent", also referred to as a filler, is used primarily to increase the weight and volume of the tablet. The addition of a diluent not only ensures a certain volume, but also reduces the dose deviation of the main ingredients and improves the compression moldability, etc., of the drug. When a drug in tablet form contains oily components, an absorbent is necessarily added to absorb the oily components so as to maintain a "dry" state that facilitates the preparation of tablets.

The term "naked antibody" refers to an antibody that is not conjugated to a heterologous moiety (e.g., a cytotoxic drug).

## BRIEF DESCRIPTION OF THE DRAWING

FIG. 1 shows the bystander killing activity of ADC-1 against a mixture of EBC-1 cells and SW480 cells.

## DETAILED DESCRIPTION

### Examples

The present disclosure is further described below with reference to examples, but these examples are not intended to limit the scope of the present disclosure. In the examples of the present disclosure, experimental methods without specific conditions specified were generally conducted under conventional conditions, such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

### Example 1. Design and Preparation of Antibody-Drug Conjugates

#### 1.1. Anti-c-Met antibody

The following antibody Ab-10 can be prepared using a conventional preparation method for antibodies, for example, through the following steps: vector construction, then transfection of eukaryotic cells such as HEK293 cells (Life Technologies Cat. No. 11625019), expression, separation, and purification.

**The sequence of the Ab-10 humanized antibody is shown below:**

Heavy chain:

QVQLVESGGGVVQPGRSLRLSCAASGFSLSNYGVHWVRQAPGKGLEWLAVIW
SGGSTNYAAAFVSRLTISKDNSKNTVYLQMNSLRAEDTAVYYCARNHDNPYN
YAMDYWGQGTTVTVSSASTKGPSVFPLAPCSRSTSESTAALGCLVKDYFPEPVT
VSWNSGALTSGVHTFPAVLQSSGLYSLSSVVTVPSSNFGTQTYTCNVDHKPSNT
KVDKTVERKCCVECPPCPAPPVAGPSVFLFPPKPKDTLMISRTPEVTCVVVDVS
HEDPEVQFNWYVDGVEVHNAKTKPREEQFNSTFRVVSVLTVVHQDWLNGKE

YKCKVSNKGLPAPIEKTISKTKGQPREPQVYTLPPSREEMTKNQVSLTCLVKGF
YPSDIAVEWESNGQPENNYKTTPPMLDSDGSFFLYSKLTVDKSRWQQGNVFSC
SVMHEALHNHYTQKSLSLSPGK

SEQ ID NO: 18

Light chain:

DIVLTQSPDSLAVSLGERATINCRADKSVSTSTYNYLHWYQQKPGQPPKLLIYL
ASNLASGVPDRFSGSGSGTDFTLTISSLQAEDVAVYYCQHSRDLPPTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC

SEQ ID NO: 19.

1.2. Preparation of compound 1

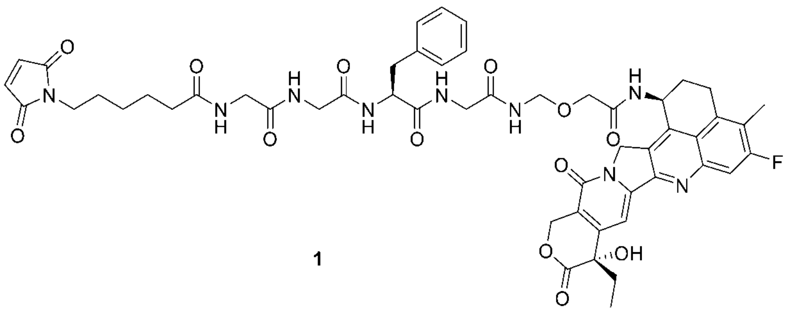

1

.

The title compound 1 was synthesized with reference to the method provided in "Example 58 on page 163 of the specification of Patent No. CN104755494A".

**1.3. Preparation of compound 9A and compound 9B**

*N*-((2*R*,10*S*)-10-Benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4":6,7]indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **9-A**

*N*-((2*S*,10*S*)-10-Benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **9-B**

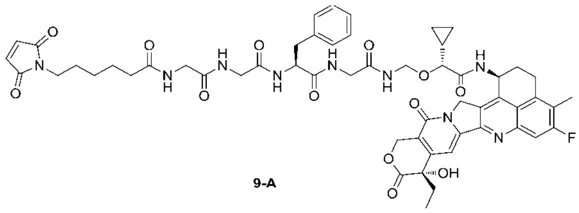
9-A

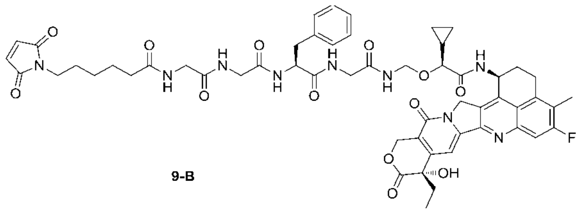
9-B

Step 1

Benzyl 2-cyclopropyl-2-hydroxyacetate **9a**

**2a** (1.3 g, 11.2 mmol; prepared using the method disclosed in Patent Application No. "WO2013/106717") was dissolved in 50 mL of acetonitrile, and potassium carbonate (6.18 g, 44.8 mmol), benzyl bromide (1.33 mL, 11.2 mmol), and tetrabutylammonium iodide (413 mg, 1.1 mmol) were sequentially added. The reaction mixture was stirred at room temperature for 48 h and filtered through diatomaceous earth, and the filter cake was rinsed with ethyl acetate (10 mL). The filtrates were combined and concentrated under reduced pressure, and the resulting residue was purified by silica gel column chromatography with developing solvent system C to give the title product **9a** (2 g, yield: 86.9%).

Step 2

Benzyl 10-cyclopropyl-1-(9*H*-fluoren-9-yl)-3,6-dioxo-2,9-dioxa-4,7-diazaundecan-11-oate **9b**

**9a** (120.9 mg, 0.586 mmol) and **8b** (180 mg, 0.489 mmol, prepared using the method disclosed in Patent Application No. "CN105829346A") were added to a reaction flask, and 4 mL of tetrahydrofuran was added. The system was purged with argon three times and cooled to 0-5 °C in an ice-water bath, and potassium tert-butoxide (109 mg, 0.98 mmol) was added. The ice bath was removed, and the mixture was warmed to room temperature and stirred for 40 min. 10 mL of iced water was added, and extraction was performed with ethyl acetate (20 mL × 2) and chloroform (10 mL × 5). The organic phases were combined and concentrated. The resulting residue was dissolved in 4 mL of dioxane. 2 mL of water was added, and sodium bicarbonate (49.2 mg, 0.586 mmol) and 9-fluorenylmethyl chloroformate (126 mg, 0.49 mmol) were added. The mixture was stirred at room temperature for 2 h. 20 mL of water was added, and extraction was performed with ethyl acetate (10 mL × 3). The organic phase was washed with a saturated sodium chloride solution (20 mL), dried over anhydrous sodium sulfate, and filtered. The filtrate was concentrated under reduced pressure. The resulting residue was purified by silica gel column chromatography with developing solvent system C to give the title product **9b** (48 mg, yield: 19%).

MS m/z (ESI): 515.0 [M+1].

Step 3

10-Cyclopropyl-1-(9*H*-fluoren-9-yl)-3,6-dioxo-2,9-dioxa-4,7-diazaundecan-11-oic acid **9c**

**9b** (20 mg, 0.038 mmol) was dissolved in 4.5 mL of a solvent mixture of tetrahydrofuran and ethyl acetate (V:V = 2:1), and palladium on carbon (12 mg, 10% loading, dry) was added. The system was purged with hydrogen three times, and the mixture was stirred at room temperature for 1 h. The reaction mixture was filtered through diatomaceous earth, and the filter cake was rinsed with ethyl acetate. The filtrate was concentrated to give the crude title product **9c** (13 mg). The product was directly used in the next step without purification.

MS m/z (ESI): 424.9 [M+1].

Step 4

(9*H*-Fluoren-9-yl)methyl (2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-oxoethoxy)methyl)amino)-2-oxoethyl)carbamate **9d**

**1b** (10 mg, 18.8 μmol) was added to a reaction flask, and 1 mL of *N,N*-dimethylformamide was added. The system was purged with argon three times and cooled to 0-5 °C in an ice-water bath, and a drop of triethylamine was added. Crude **9c** (13 mg, 30.6 μmol) was added, and 4-(4,6-dimethoxy-1,3,5-triazin-2-yl)-4-methylmorpholinium chloride (16.9 mg, 61.2 μmol) was added. The mixture was stirred in the ice bath for 40 min. 10 mL of water was added, and extraction was performed with ethyl acetate (10 mL × 3). The organic phases were combined, washed with a saturated sodium chloride solution (10 mL × 2), dried over anhydrous sodium sulfate, and filtered. The filtrate was concentrated under reduced pressure. The resulting residue was purified by thin-layer chromatography with developing solvent system B to give the title product **9d** (19 mg, yield: 73.6%).

MS m/z (ESI): 842.1 [M+1].

Step 5

2-((2-Aminoacetylamino)methoxy)-2-cyclopropyl-*N*-((1*S*,9*S*)-9-ethyl-5-fluoro-9-hydroxy-4-methyl-10,13-dioxo-2,3,9,10,13,15-hexahydro-1*H,*12*H*-benzo[*de*]pyrano[3',4':6,7]indolizino[1,2-*b*]quinolin-1-yl)acetamide **9e**

**9d** (19 mg, 22.6 μmol) was dissolved in 2 mL of dichloromethane, and 1 mL of diethylamine was added. The mixture was stirred at room temperature for 2 h. The reaction mixture was concentrated under reduced pressure. 1 mL of toluene was added, and the mixture was concentrated under reduced pressure; the procedures were repeated twice. The residue was triturated with 3 mL of n-hexane, and the triturate was left to stand. The supernatant was then removed, and the solid was kept. The solid residue was concentrated under reduced pressure and dried using an oil pump to give the crude title product **9e** (17 mg). The product was directly used in the next step without purification. MS m/z (ESI): 638.0 [M+18].

Step 6

*N*-((2*R*,10*S*)-10-Benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **9-A**

*N*-((2*S*,10*S*)-10-Benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **9-B**

Crude **9e** (13.9 mg, 22.4 μmol) was dissolved in 0.6 mL of *N,N*-dimethylformamide. The system was purged with argon three times and cooled to 0-5 °C in an ice-water bath. A solution of **8g** (21.2 mg, 44.8 μmol, prepared using the method disclosed in Patent Application No. "EP2907824") in 0.3 mL of *N,N*-dimethylformamide was added, and 4-(4,6-dimethoxy-1,3,5-triazin-2-yl)-4-methylmorpholinium chloride (18.5 mg, 67.3 μmol) was added. The mixture was stirred in an ice bath for 10 min. The ice bath was removed, and the mixture was warmed to room temperature and stirred for 1 h. The reaction yielded compound **9.** The reaction mixture was purified by high performance liquid chromatography (separation conditions: chromatography column: XBridge Prep C18 OBD 5 μm 19 × 250 mm; mobile phase: A-water (10 mmol of $NH_4OAc$):B-acetonitrile, gradient elution, flow rate: 18 mL/min). The corresponding fractions were collected and concentrated under reduced pressure to give the title products (9-A: 2.4 mg, 9-B: 1.7 mg). MS m/z (ESI): 1074.4 [M+1].

Single-configuration compound 9-A (shorter retention time):
UPLC analysis: retention time: 1.14 min, purity: 85% (chromatography column: ACQUITY UPLC BEHC18 1.7 μm 2.1 × 50 mm, mobile phase: A-water (5 mmol $NH_4OAc$), B-acetonitrile).

$^1$H NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 1H), 4.54-4.40 (m, 2H), 3.76-3.64 (m, 4H), 3.62-3.48 (m, 2H), 3.20-3.07 (m, 2H), 3.04-2.94 (m, 1H), 2.80-2.62 (m, 1H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

Single-configuration compound 9-B (longer retention time):
UPLC analysis: retention time: 1.16 min, purity: 89% (chromatography column: ACQUITY UPLC BEHC18 1.7 μm 2.1 × 50 mm, mobile phase: A-water (5 mmol $NH_4OAc$), B-acetonitrile).

$^1$H NMR (400 MHz, DMSO-$d_6$): δ 8.68-8.60 (m, 1H), 8.58-8.50 (m, 1H), 8.32-8.24 (m, 1H), 8.13-8.02 (m, 2H),

8.02-7.94 (m, 1H), 7.82-7.75 (m, 1H), 7.31 (s, 1H), 7.26-7.13 (m, 3H), 6.99 (s, 1H), 6.55-6.48 (m, 1H), 5.60-5.50 (m, 1H), 5.41 (s, 2H), 5.35-5.15 (m, 2H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 4H), 0.91-0.79 (m, 3H), 0.53-0.34 (m, 4H).

**1.4. Preparation of antibody-drug conjugate-1 (ADC-1)**

Antibody-drug conjugate-1 (ADC-1) of the following structure was prepared. The Ab in ADC-1 was Ab-10.

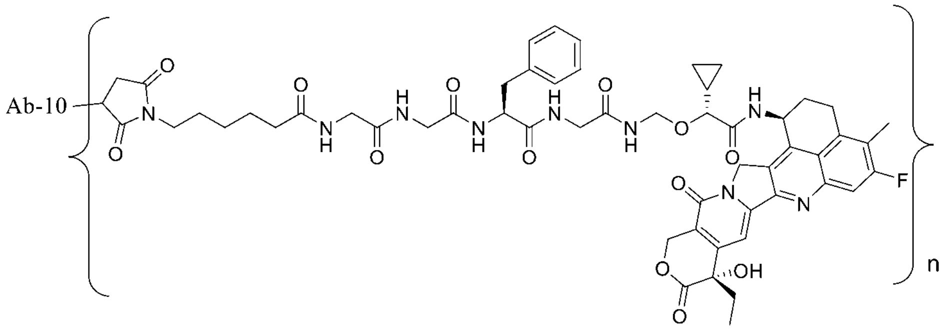

An appropriate amount of an aqueous histidine-histidine hydrochloride buffer (10 mM histidine-histidine hydrochloride, pH = 5.8) was added to antibody Ab-10 at 37 °C, and an appropriate amount of 200 mM EDTA (pH 6.3) was then added to make an antibody concentration of 15 g/L. A prepared aqueous solution (10 mM, 14 eq) of tris(2-carboxyethyl) phosphine hydrochloride (TCEP-HCl) was then added. The mixture was shaken at 25 °C for 22-24 h, and the reaction was stopped.

Compound 9-A (11 eq) was dissolved in dimethyl sulfoxide, and the solution was added to the above reaction mixture. The mixture was shaken on a water bath shaker at 25 °C for 2 h, and the reaction was stopped. The reaction mixture was purified by desalting with a Capto S Impact cation chromatography column (eluent: a 50 mM HAc-NaAc buffer (pH 5.5), containing 390 mM NaCl) to give an exemplary product of general formula ADC-1, ADC-1 in the HAc-NaAc buffer. The product was refrigerated at 4 °C.

The target DAR value was: n = 5.3. Through RP-HPLC analysis, the average drug loading was calculated. The found DAR value was: n = 4.7-5.9.

**1.5. Preparation of antibody-drug conjugate-2 (ADC-2)**

With Ab-10 and compound 1, antibody-drug conjugate-2 (ADC-2) of the following structure was prepared with reference to the preparation method described in step 1.4 above. The Ab in ADC-2 was Ab-10, and the target DAR value was 6:

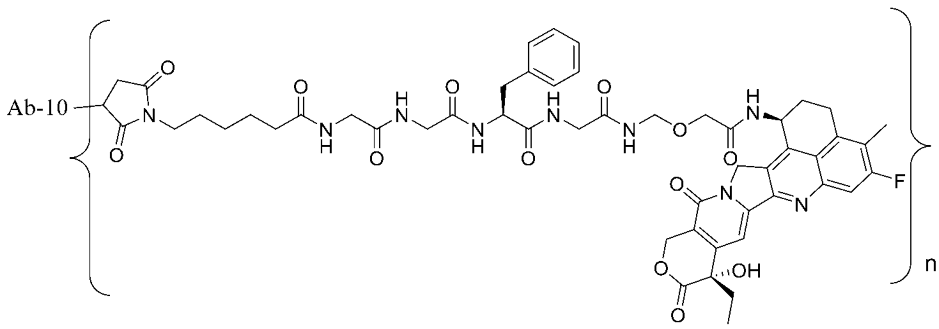

**Example 2. Binding Activity of Antibody-Drug Conjugate to c-Met Protein**

The affinities of the antibody-drug conjugate ADC-1 for c-Met proteins of different species (human and cynomolgus monkey) were determined using Biacore technology. A Protein A sensor chip was placed into a Biacore 8K instrument. HBS-EP+ was used as an assay buffer solution. Each cycle involved ligand capture, analyte injection, and regeneration. The instrument temperature was set to 25 °C. Solutions of ADC-1 and Ab-10 were prepared as ligands using

the HBS-EP+ buffer solution. The ligands were captured using Protein A in a detection channel (Fc2) at a flow rate of 10 μL/min for 30 s. Solutions of human, cynomolgus monkey, and mouse c-Met proteins with different concentrations were prepared as analytes using the HBS-EP+ buffer solution, and the analytes were injected into the reference channel and detection channels (Fc1&2) of the chip at a flow rate of 30 μL/min. The analytes were serially diluted 2-fold. The experimental parameters were set. Finally, the chip was regenerated using Glycine 1.5 at a flow rate of 30 μL/min for 30 s. SPR signals were collected and saved using Biacore 8K Control Software, and data were further processed using Biacore 8K Evaluation analysis software. The corresponding reference channel (Fc1) signal values were subtracted from the detection channel (Fc2) signal values to obtain a corrected signal curve. Affinity evaluations used a double subtraction method. A zero concentration cycle with the same parameter settings (Sample 0) was added before the sample cycle, and the zero concentration cycle was subtracted from the sample cycle (Sample - Sample 0) to obtain a doubly corrected signal curve. Affinity kinetic curves were fitted using the 1:1 Langmuir binding model, and $K_D$ values were calculated. The affinities of the antibody or antibody-drug conjugate for the proteins are shown in Table 2.

The experimental results show that ADC-1 and its naked antibody Ab-10 have relatively strong affinities for human and cynomolgus monkey c-Met proteins.

**Table 2. The affinity of ADC-1 for human or cynomolgus monkey c-Met**

| Antigen | Test drug | KD (M) |
|---|---|---|
| Human c-Met | ADC-1 | 2.69E-08 |
| | Ab-10 | 2.62E-08 |
| Cynomolgus monkey c-Met | ADC-1 | 4.85E-08 |
| | Ab-10 | 4.79E-08 |

### Example 3. Evaluation of Ability of Antibody-Drug Conjugate to Inhibit Tumor Cell Proliferation

The effect of the antibody-drug conjugate on the *in vitro* proliferation of different tumor cell lines was determined using the CellTiter Glo method.

MKN45 cells, NCI-H1993 cells, NCI-H441 cells, and NCI-N87 cells were each cultured in an RPMI1640 complete culture medium containing 10% FBS. EBC-1 cells were cultured in an MEM complete culture medium containing 10% FBS. SW480 cells were cultured in an L-15 culture medium containing 10% FBS.

In the experiment, after digestion with 0.25% trypsin-EDTA, centrifugation was performed at 400 g for 5 min, and the supernatant was discarded. The cell lines were resuspended in their respective culture media. The cell density was adjusted with the corresponding culture medium. In the assay system, the serum concentration of NCI-H1299 cells was 2%, and the serum concentration of the other cells was 5%. In 96-well plates, 100 μL of cell suspension was added to each well; NCI-H1993 cells, EBC-1 cells, NCI-H441 cells, MKN-45 cells, NCI-N87 cells, and SW480 cells were plated at 1000 cells/well, NCI-H1299 cells and HCT116 cells were plated at 500 cells/well, and Hs 746T cells were plated at 1500 cells/well. The plates were incubated overnight in an incubator (37 °C, 5% $CO_2$). ADC-1, its naked antibody Ab-10, and its toxin moiety were serially diluted and added to cell culture media. After 6 days of cell culture, an assay was performed using a Cell Counting-Lite 2.0 Luminescent Cell Viability Assay kit (VAZYME, DD1101-02) according to the instructions. Chemiluminescence was measured using a microplate reader (BMG, CLARIOstar plus), and data analysis was performed using GraphPad Prism (version 9) software.

The experimental results are shown in Table 3. The ADC-1 of the present disclosure exhibited stronger inhibitory activity against gastric cancer MKN-45 cells (which highly express c-Met) than its naked antibody and toxin. The results show that the ADC-1 of the present disclosure exhibited relatively strong tumor proliferation inhibiting activity. Among the lung cancer cells shown in Table 3, NCI-H1993 and EBC-1 highly express c-Met, NCI-H441 moderately expresses c-Met, and NCI-H1299 lowly expresses c-Met. Among the gastric cancer cells, Hs 746T and MKN-45 highly express c-Met, and NCI-N87 lowly expresses c-Met. Both colon cancer HTC116 and SW480 cells lowly express c-Met.

As shown in Table 3, ADC-1 also exhibited relatively strong proliferation inhibiting activity against other tumor cell lines that highly express c-Met (NCI-H1993, EBC-1, and Hs 746T). In particular, lung cancer EBC-1 cells were more sensitive to ADC-1. For the moderate-expression lung cancer cell line NCI-H441, the anti-c-Met naked antibody showed extremely weak activity, whereas ADC-1, which carries a toxin, showed strong proliferation inhibiting activity. However, despite the relatively weak inhibition of the cell lines that lowly express c-Met (NCI-H1299, NCI-N87, HCT116, and SW480), its toxin moiety still exhibited a significant inhibitory effect on these cell lines that lowly express c-Met.

The above results show that the ADC-1 of the present disclosure exhibited specific targeting and relatively strong inhibitory activity against the lung cancer cell lines NCI-H1993 and EBC-1 and the gastric cancer cell line Hs 746T, which highly express c-Met; for the lung cancer cell line NCI-H441, the anti-c-Met antibody exhibited extremely weak inhibitory activity, whereas ADC-1 still exhibited good inhibitory activity. The toxicity to non-specific cells that lowly express c-Met

(e.g., normal cells) was significantly smaller.

**Table 3. The $IC_{50}$ values (nM) for the inhibition of the proliferation of tumor cell lines by ADC-1**

| Tumor type | Cell strain | c-Met expression | ADC-1 | Ab-10 | Toxin moiety |
|---|---|---|---|---|---|
| Lung cancer | NCI-H1993 | High | 0.181±0.033 | 0.203±0.021 | 3.810±0.258 |
| | EBC-1 | High | 0.065±0.011 | 0.051±0.005 | 0.580±0.087 |
| | NCI-H441 | Moderate | 0.480±0.110 | >200 | 0.488±0.056 |
| | NCI-H1299 | Low | >200 | >200 | 1.107±0.102 |
| Gastric cancer | Hs 746T | High | 0.125±0.049 | 0.134±0.020 | 2.897±0.522 |
| | MKN-45 | High | 0.055±0.009 | 0.176±0.034 | 0.507±0.051 |
| | NCI-N87 | Low | >200 | >200 | 1.223±0.245 |
| Colon cancer | HCT116 | Low | 23.930±5.244 | >200 | 0.306±0.080 |
| | SW480 | Low | >200 | >200 | 1.497±0.487 |

**Example 4. Endocytosis Ability of Antibody-Drug Conjugate**

To assess the time dependence of endocytosis after the antibody-drug conjugate binds to c-Met-positive cells at a single concentration, human lung cancer EBC-1 cells were used, and an ADC-1 group, a naked antibody Ab-10 group, a Teli-VcMMAE group, and a naked antibody ABT-700 group were set. Teli-VcMMAE (namely ABBV-399, in which the toxin is MMAE) and its naked antibody ABT-700 were prepared in-house based on the telisotuzumab vedotin structure disclosed on page 319 of WHO Drug Information, Vol. 30, No. 2, 2016.

EBC-1 cells were cultured (in an MEM culture medium containing 10% FBS), washed once with PBS, and then digested with TrypLE™ Express Enzyme (Gibco, 12605-010) to form a single cell suspension, and the suspension was centrifuged at 400 g for 5 min. The cell density was adjusted to $2 \times 10^6$ cells/mL using a FACS buffer (prepared with PBS 1×, pH 7.2, and 2% FBS). The suspension was added to a 96-well round-bottom plate at 50 μL/well. Groups were set as follows: endocytosis for 0 h, endocytosis for 0.5 h, endocytosis for 2 h, endocytosis for 4 h, and endocytosis for 24 h. A 40 nM (2×) dilution of an antibody or antibody-drug conjugate was added at 50 μL/well. The assay groups included ADC-1, the naked antibody Ab-10, Teli-VcMMAE, and the naked antibody ABT-700. A final concentration of 20 nM was achieved. The plate was incubated at 4 °C for 60 min. After the incubation, the plate was centrifuged at 400 g for 5 min, the supernatant was discarded, and the plate was washed twice with 200 μL of FACS Buffer per well. The cells were resuspended in a culture medium and subjected to 0 h, 0.5 h, 2 h, 4 h, or 24 h of endocytosis in an incubator (37 °C, 5% $CO_2$). After different endocytosis periods, the cells were digested with TrypLE™ Express Enzyme to form a single cell suspension. After 1 wash with 200 μL of FACS buffer, a secondary antibody (Alexa Fluor® 647 AffiniPure Mouse Anti-Human IgG(H+L), 1:500, Jackson, 209-605-008) diluted in FACS buffer was added, and the plate was incubated in a dark place at 4 °C for 30 min. The plate was centrifuged at 400 g for 5 min, the supernatant was discarded, and the plate was washed twice with 200 μL of FACS buffer per well. The cells were resuspended in 50 μL of FACS buffer, and 50 μL of 4% paraformaldehyde was then added for fixation. After thorough mixing, the plate was left to stand at 4 °C in a dark place. After all samples were collected, Alexa Fluor® 647 fluorescence signals were detected using a flow cytometer. Data were analyzed using Flowjo software, and gMFI values were calculated. Endocytosis ratio = (0-h endocytosis gMFI - n-h endocytosis gMFI)/0-h endocytosis gMFI. The results are shown in Table 4.

Table 4. Evaluation of endocytosis performance

| Endocytosis ratio% | 0 h | 0.5 h | 2 h | 4h | 24 h |
|---|---|---|---|---|---|
| Ab-10 Naked antibody | 0.000 | 24.516 | 62.579 | 83.032 | 98.447 |
| ABT-700 Control antibody | 0.000 | 5.537 | 29.293 | 54.152 | 97.753 |
| ADC-1 | 0.000 | 30.186 | 61.043 | 80.560 | 98.189 |
| Teli-VcMMAE | 0.000 | 4.361 | 31.734 | 58.026 | 97.339 |

The results show that during the detection period, the ratio of endocytosis of ADC-1 and its naked antibody Ab-10 by EBC-1 cells gradually increased with incubation time, demonstrating a dependence on time. Moreover, at 4 h, both ADC-1 and its naked antibody (the endocytosis ratio was about 80%) showed higher endocytosis ratios than Teli-VcMMAE

and its naked antibody ABT-700 (the endocytosis ratio was about 55%), indicating that ADC-1 and its naked antibody can be endocytosed rapidly by the target cell.

**Example 5. Bystander Killing Effect of Antibody-Drug Conjugate**

EBC-1 cells (which highly express c-Met) and SW480 cells (which lowly express c-Met) were cultured in an MEM culture medium containing 10% FBS and an L-15 culture medium containing 10% FBS, respectively, and trypsinized to form single cell suspensions, and the suspensions were centrifuged at 400 g for 5 min. The cells were washed once with PBS, and the cell density was then adjusted to $1 \times 10^6$ cells/mL with PBS. EBC-1 and SW480 cells were labeled with CellTrace™ Violet (Thermo, C34557) and CellTrace™ CFSE (Thermo, C34554A), respectively, at a ratio of 1 μL of dye per mL of suspension in PBS at 37 °C for 15 min. After the labeling, the dyeing was stopped with a complete culture medium. The mixtures were centrifuged at 400 g for 5 min, and the cells were washed twice with PBS. EBC-1 cells and SW480 cells were each resuspended in a 1:1 mixture of an MEM culture medium containing 5% FBS and an L15 culture medium, and the suspensions were inoculated into 24-well plates. In the mixed culture group, each well contained $9 \times 10^4$ EBC-1 cells and $1.5 \times 10^4$ SW480 cells. In the SW480-alone culture group, each well contained $1.5 \times 10^4$ cells. The culture system was 500 μL in volume. Adherent culture was performed overnight in an incubator (5% $CO_2$, 37 °C). The next day, 2× ADC-1 was added to the 24-well plates to achieve final concentrations of 0.5 nM, 5 nM, and 50 nM. An equal volume of fresh culture medium was added to the control group. The plates were incubated in a cell incubator for 72 h. After 72 h, the cells were washed once with PBS and trypsinized to form single cell suspensions, and the cells were counted using a cell counter. The ratio of CTV-positive cells to CFSE-positive cells was determined using a flow cytometer (BD Celesta Flow cytometer), and the number of EBC-1 and SW480 cells per well was calculated. Plots were generated using GraphPad Prism9, and statistical analysis of data was performed using Tukey's multiple comparisons test.

The experimental results are shown in FIG. 1. When SW480 cells, which lowly express c-Met, were cultured alone, the cell count was substantially unaffected by the varying concentrations of ADC-1. When co-cultured with EBC-1 cells, which highly express c-Met, the SW480 cell count decreased with the increasing ADC-1 dose.

The result shows that the ADC-1 of the present disclosure exhibited a definite bystander killing effect; the ADC did not kill SW480 cells, which lowly express c-Met, when the cells were cultured alone; however, when EBC-1 cells, which highly express c-Met, were mixed with SW480 cells, which lowly express c-Met, the ADC also exhibited a killing effect on the cells that lowly express c-Met.

**Example 6. Evaluation of *In Vivo* Efficacy in Mouse Xenograft Tumor Model of Human Lung Cancer NCI-H441 Cells**

Balb/c Nude mice were subcutaneously inoculated with NCI-H441 cells ($5 \times 10^6$). When the tumor volume grew to 140 $mm^3$ on average, the mice were randomly divided into 4 groups of 6 according to tumor size and body weight: a vehicle control group and three dose groups for ADC-1 (1, 3, and 10 mg/kg). The day of grouping was defined as D0. The vehicle (0.9% NaCl) or the drug ADC-1 (1, 3, and 10 mg/kg) was administered twice (D0 and D7) by intraperitoneal injection. After the grouping, the tumor volume was measured twice weekly, and the mouse body weight was recorded for 21 days.

Data were recorded using Excel statistical software: average values were calculated using avg; SD values were calculated using STDEV; SEM values were calculated using STDEV/SQRT (number of animals per group); plots were generated using GraphPad Prism software, and statistical analysis of data was performed using Two-way ANOVA or One-way ANOVA.

Tumor volume was calculated as follows:

$$\text{tumor volume (V)} = (\text{length} \times \text{width}^2)/2.$$

$$\text{Relative tumor volume RTV} = V_t/V_0 \times 100\ (\%),$$

where $V_0$ represents the tumor volume measurement at the time of grouping and administration, and $V_t$ represents the tumor volume at the time of each measurement.

$$\text{Relative tumor proliferation rate T/C}\ (\%) = (T_{RTV}/C_{RTV}) \times 100\%,$$

where T and C represent the tumor volumes of the treatment group and the control group at the end of the experiment; $T_0$ and $C_0$ represent the tumor volumes at the beginning of the experiment.

$$\text{Tumor growth inhibition rate TGI (\%)} = 1 - \text{T/C (\%)}.$$

The experimental results are shown in Table 5. Compared to the vehicle control group, 1, 3, and 10 mg/kg ADC-1 all exhibited significant tumor inhibiting effects, with TGI% values of 92.91% , 93.70%, and 95.71%, respectively. Moreover, the experimental animals did not significantly lose weight during the administration period. This indicates that the test drug exhibited a good therapeutic effect on tumors. Even at the dose of 1 mg/kg, the test drug exhibited a tumor growth inhibition rate of 92.91%. Moreover, the test drug did not cause significant toxic or side effects on the experimental animals, suggesting good tolerability and safety.

Table 5. The inhibition of the growth of a human lung cancer NCI-H441 nude mouse subcutaneous xenograft tumor model

| Test substance | Dose (mg/kg) | Mean tumor volume ($mm^3$, Mean ± SEM) | | T/C (%) D21 | TGI (%) D21 |
|---|---|---|---|---|---|
| | | D0 | D21 | | |
| Vehicle | - | 140±9 | 1143±190 | - | - |
| ADC-1 | 1 | 140±7 | 81±20*** | 7.09 | 92.91 |
| ADC-1 | 3 | 140±9 | 72±9*** | 6.30 | 93.70 |
| ADC-1 | 10 | 140±9 | 49±4*** | 4.29 | 95.71 |

Note: All groups were compared to the vehicle control group; $^{*}p < 0.05$, $^{**}p < 0.01$, $^{***}p < 0.001$.

**Example 7. Evaluation of *In Vivo* Efficacy in Human Gastric Cancer MKN-45 Nude Mouse Xenograft Tumor Model**

Female Balb/c Nude mice were subcutaneously inoculated with $5 \times 10^6$ MKN-45 cells to establish a subcutaneous tumor model. When the tumors grew to about 117 $mm^3$, the mice were randomly divided into 7 groups of 6 according to tumor size and mouse body weight: a vehicle control group, a naked antibody Ab-10 group (10 mg/kg), three dose groups for ADC-1 (1, 3, and 10 mg/kg), a reference drug Teli-VcMMAE group (3 mg/kg), and an IgG2-drug control group (3 mg/kg, an IgG2 antibody conjugated to a linker and a toxic drug which are the same as those in ADC-1, DAR 5.3). Each group was given a single (D0) intraperitoneal injection. The body weight and tumor size of the animals were measured twice weekly during the experiment.

Tumor volume was calculated as follows:

$$\text{tumor volume} = (\text{length} \times \text{width}^2)/2.$$

Relative tumor change rate $\Delta T/\Delta C$ (%) = (final tumor volume of treatment group - initial tumor volume of treatment group)/(final tumor volume of vehicle group - initial tumor volume of vehicle group) × 100%.

$$\text{Tumor growth inhibition rate TGI\%} = (1 - \Delta T/\Delta C) \times 100\%.$$

Data were recorded using Excel statistical software: average values were calculated using avg; SD values were calculated using STDEV; SEM values were calculated using STDEV/SQRT (number of animals per group); plots were generated using GraphPad Prism software, and statistical analysis of data was performed using Two-way ANOVA or One-way ANOVA.

The experimental results are shown in Table 6. Compared to the vehicle control group, both 3 mg/kg ADC-1 and 10 mg/kg ADC-1 significantly inhibited the growth of MKN-45 tumors *in vivo*, with TGI% values of 94.72% and 103.56%, respectively. However, the same dose of the reference drug Teli-VcMMAE (3 mg/kg) did not show significant antitumor activity, with a TGI% of 40.28%. Similarly, even at the dose of 10 mg/kg, the naked antibody Ab-10 and IgG2-toxin did not show significant antitumor activity, with TGI% values of 48.34% and 23.42%, respectively. This indicates that the test drug ADC-1 exhibited a good antitumor effect. The test drug ADC-1 exhibited significantly better efficacy than the reference drug Teli-VcMMAE and the naked antibody Ab-10 when they were administered at the same dose, and ADC-1 did not produce significant toxic or side effects on the experimental animals, suggesting good tolerability and safety. Interestingly, even the low dose (3 mg/kg) of ADC-1 achieved the same tumor inhibiting effect as 10 mg/kg, and both test doses were well tolerated by tumor-bearing mice, suggesting that ADC-1 has a wide therapeutic window.

Table 6. The inhibition of the growth of a human gastric cancer MKN-45 nude mouse subcutaneous xenograft tumor model

| Test substance | Dose (mg/kg) | Mean tumor volume ($mm^3$, mean ± SEM) | | TGI (%) D23 |
|---|---|---|---|---|
| | | D0 | D23 | |
| Vehicle | - | 116 ± 2 | 2189 ± 282 | - |
| Ab-10 | 10 | 117± 3 | 1187 ± 102 | 48.34% |
| ADC-1 | 1 | 117±2 | 1396±178 | 38.26% |
| ADC-1 | 3 | 117± 3 | 226±46** | 94.72% |
| ADC-1 | 10 | 116 ± 2 | 43±4*** | 103.56% |
| Teli-VcMMAE | 3 | 117± 3 | 1354 ± 327 | 40.28% |
| IgG2-toxin | 10 | 117±2 | 1704 ± 158 | 23.42% |

Note: All groups were compared to the vehicle control group; $*p < 0.05$, $**p < 0.01$, $***p < 0.001$.

**Example 8. Evaluation of *In Vivo* Efficacy in Human Gastric Cancer GA6831 PDX Nude Mouse Xenograft Tumor Model**

BALB/c nude mice were subcutaneously inoculated with human gastric cancer GA6831 tumor blocks (tumor tissue was harvested from HuPrime® gastric cancer xenograft model GA6831 tumor-bearing mice and cut into tumor blocks with a diameter of 2-3 mm) to establish a human gastric cancer subcutaneous xenograft tumor model. When the tumors grew to about 151 $mm^3$ on average, the mice were randomly divided into 4 groups of 7 according to tumor size and body weight: a vehicle control group and three dose groups for ADC-1 (1, 3, and 10 mg/kg). The day of grouping was defined as D0.

The vehicle (0.9% NaCl) or the drug ADC-1 (1, 3, and 10 mg/kg) was administered by intraperitoneal injection on D0, D7, and D14. After the grouping, the tumor volume and mouse body weight were measured twice weekly.

Data were recorded using Excel statistical software: average values were calculated using avg; SD values were calculated using STDEV; SEM values were calculated using STDEV/SQRT (number of animals per group); plots were generated using GraphPad Prism software, and statistical analysis of data was performed using Two-way ANOVA or One-way ANOVA.

Tumor volume was calculated as follows:

$$\text{tumor volume (V)} = (\text{length} \times \text{width}^2)/2.$$

Relative tumor proliferation rate ΔT/ΔC (%) = (final tumor volume of treatment group - initial tumor volume of treatment group)/(final tumor volume of vehicle group - initial tumor volume of vehicle group) × 100%.

$$\text{Tumor growth inhibition rate TGI\%} = (1 - \Delta T/\Delta C) \times 100\%.$$

The experimental results are shown in Table 7. Compared to the vehicle control group, 1 mg/kg, 3 mg/kg, and 10 mg/kg ADC-1 all significantly inhibited the growth of the human HuPrime® gastric cancer GA6831 subcutaneous xenograft *in vivo* model, with TGI% values of 65.65%, 73.56%, and 91.70%, respectively. Moreover, the experimental animals did not significantly lose weight during the administration period. This indicates that the test drug exhibited a good therapeutic effect on tumors. Moreover, the test drug did not cause significant toxic or side effects on the experimental animals, suggesting good tolerability and safety.

Table 7. The inhibition of the growth of a human gastric cancer GA6831 PDX nude mouse subcutaneous xenograft tumor model

| Test substance | Dose (mg/kg) | Mean tumor volume ($mm^3$, Mean ± SEM) | | T/C (%) D21 | TGI (%) D21 |
|---|---|---|---|---|---|
| | | D0 | D21 | | |
| Vehicle | | 151.47 ± 10.18 | 1699.82 ± 190.40 | | |
| ADC-1 | 1 | 151.51 ± 10.11 | 682.84 ±137.50*** | 34.35 | 65.65 |

(continued)

| Test substance | Dose (mg/kg) | Mean tumor volume ($mm^3$, Mean ± SEM) | | T/C (%) D21 | TGI (%) D21 |
|---|---|---|---|---|---|
| | | D0 | D21 | | |
| ADC-1 | 3 | 151.58 ± 10.12 | 560.82 ± 103.23*** | 26.44 | 73.56 |
| ADC-1 | 10 | 151.47 ± 10.18 | 280.07 ± 65.18*** | 8.30 | 91.70 |

Note: All groups were compared to the vehicle control group; *$p$ < 0.05, **$p$ < 0.01, ***$p$ < 0.001.

**Example 9. Evaluation of *In Vivo* Efficacy in Human Colorectal Cancer CR3150 PDX Nude Mouse Xenograft Tumor Model**

BALB/c nude mice were subcutaneously inoculated with human colorectal cancer CR3150 tumor blocks (tumor tissue was harvested from HuPrime® colorectal cancer xenograft model CR3150 tumor-bearing mice and cut into tumor blocks with a diameter of 2-3 mm) to establish a human colorectal cancer subcutaneous xenograft tumor model. When the tumors grew to about 156 $mm^3$ on average, the mice were randomly divided into 3 groups of 7 according to tumor size and body weight: a vehicle control group and two dose groups for ADC-1 (3 and 10 mg/kg). The day of grouping was defined as Day 0.

The vehicle (0.9% NaCl) or ADC-1 (3 and 10 mg/kg) was administered by intraperitoneal injection on D0 and D7. After the grouping, the tumor volume and mouse body weight were measured twice weekly.

Data were recorded using Excel statistical software: average values were calculated using avg; SD values were calculated using STDEV; SEM values were calculated using STDEV/SQRT (number of animals per group); plots were generated using GraphPad Prism software, and statistical analysis of data was performed using Two-way ANOVA or One-way ANOVA.

Tumor volume was calculated as follows:

$$\text{tumor volume (V)} = (\text{length} \times \text{width}^2)/2.$$

Relative tumor proliferation rate ΔT/ΔC (%) = (final tumor volume of treatment group - initial tumor volume of treatment group)/(final tumor volume of vehicle group - initial tumor volume of vehicle group) × 100%.

$$\text{Tumor growth inhibition rate TGI\%} = (1 - \Delta T/\Delta C) \times 100\%.$$

The experimental results are shown in Table 8. Compared to the vehicle control group, both 3 and 10 mg/kg ADC-1 significantly inhibited the growth of the human HuPrime® colorectal cancer CR3150 subcutaneous xenograft *in vivo* model, with TGI% values of 70.58% and 106.95%, respectively. Moreover, during the administration period, the experimental animals did not significantly lose weight, nor were accidental mouse deaths observed. This indicates that the test drug exhibited a good therapeutic effect on tumors. Moreover, the test drug did not cause significant toxic or side effects on the experimental animals, suggesting good tolerability and safety.

Table 8. The inhibition of the growth of a human colorectal cancer CR3150 PDX nude mouse subcutaneous xenograft tumor model

| Test substance | Dose (mg/kg) | Mean tumor volume ($mm^3$) | | T/C (%) D21 | TGI (%) D21 |
|---|---|---|---|---|---|
| | | D0 | D21 | | |
| Vehicle | - | 156.53 ± 8.24 | 1480.34 ± 417.42 | - | - |
| ADC-1 | 3 | 156.41 ± 7.98 | 545.87 ± 339.19*** | 29.42 | 70.58 |
| ADC-1 | 10 | 156.10 ± 7.76 | 64.07 ± 26.44*** | -6.95 | 106.95 |

Note: All groups were compared to the vehicle control group; *$p$ < 0.05, **$p$ < 0.01, ***$p$ < 0.001.

**Example 10. Evaluation of *In Vivo* Safety in Cynomolgus Monkeys**

1) Safety of ADC-1

ADC-1 was injected intravenously into cynomolgus monkeys divided into three groups: 5, 15, and 40 mg/kg. Each group consisted of 10 animals, with an equal number of males and females. The animals were dosed once every 2 weeks for 13 consecutive weeks (7 doses in total), followed by a 4-week recovery period.

Results showed that no animals were found dead or dying throughout the study. The primary target organs of toxicity were the gastrointestinal tract and the lymphohematopoietic system. In the 40 mg/kg ADC-1 treatment group of cynomolgus monkeys, decreases in red blood cell (RBC) count, hemoglobin, hematocrit (HCT), reticulocyte (RET) count, and albumin (ALB) levels were observed. After the 4-week recovery period, no lesions were observed. These findings indicate that the highest non-severely toxic dose (HNSTD) of ADC-1 in cynomolgus monkeys was 40 mg/kg.

2) Safety of control ADC-3

ADC-12 was synthesized with reference to the method provided in Example 24 on page 61 of the specification of Patent No. CN106687480B.

ADC-12 was injected intravenously into cynomolgus monkeys divided into three groups: 1, 3, and 9 mg/kg. The animals were dosed once every 2 weeks for a total of 6 doses.

Results showed that in the 1 mg/kg ADC-12 group, the pathology of the animals only showed reduced lymphocytes in the spleen and thymus, with no other significant abnormalities observed; in the 3 mg/kg ADC-12 group, the pathological examination of the animals showed lesions in the immune and hematopoietic systems, digestive system (liver, pancreas, gastrointestinal tract), heart, kidneys, adrenal glands, etc.; in the 9 mg/kg ADC-12 group, most animals were dying or died. These results indicate an HNSTD of only 1 mg/kg.

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

**1.** An antibody-drug conjugate or a pharmaceutically acceptable salt thereof, having a structure represented by formula (I):

Ab-(L-Y-D)n formula (I)

wherein:

Ab is an anti-c-Met antibody comprising a heavy chain variable region (VH) and a light chain variable region (VL), wherein the VH comprises a HCDR1, a HCDR2, and a HCDR3 from the amino acid sequence set forth in any one of SEQ ID NOs: 10-12, and the VL comprises a LCDR1, a LCDR2, and a LCDR3 from the amino acid sequence set forth in any one of SEQ ID NOs: 13-15;
the CDRs are defined according to the Kabat, IMGT, Chothia, AbM, or Contact numbering scheme;
-L- is a linker unit that is $-L^1-L^2-L^3-L^4-$;
$L^1$ is -(succinimid-3-yl-*N*)-W-C(O)-, $-CH_2-C(O)-NR^3-W-C(O)-$, or -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$, and a chemical bond, wherein $p^1$ is an integer from 1 to 20;
$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;
$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;
$R^3$, $R^4$, and $R^5$ are identical or different and are each independently selected from the group consisting of hydrogen, alkyl, haloalkyl, deuterated alkyl, and hydroxyalkyl;

$R^6$ and $R^7$ are identical or different and are each independently selected from the group consisting of hydrogen, halogen, alkyl, haloalkyl, deuterated alkyl, and hydroxyalkyl;
Y is selected from the group consisting of -O-$(CR^aR^b)_m$-$CR^1R^2$-C(O)-, -O-$CR^1R^2$-$(CR^aR^b)_m$-, -O-$CR^1R^2$-, -NH-$(CR^aR^b)_m$-$CR^1R^2$-C(O)-, and -S-$(CR^aR^b)_m$-$CR^1R^2$-C(O)-;
$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl, and heterocyclyl; or $R^a$ and $R^b$, together with the carbon atom to which they are attached, form cycloalkyl or heterocyclyl; $R^1$ is selected from the group consisting of hydrogen, halogen, haloalkyl, deuterated alkyl, cycloalkyl, cycloalkylalkyl, alkoxyalkyl, heterocyclyl, aryl, and heteroaryl;
$R^2$ is selected from the group consisting of hydrogen, halogen, haloalkyl, deuterated alkyl, cycloalkyl, cycloalkylalkyl, alkoxyalkyl, heterocyclyl, aryl, and heteroaryl;
or $R^1$ and $R^2$, together with the carbon atom to which they are attached, form cycloalkyl or heterocyclyl;
or $R^a$ and $R^2$, together with the carbon atoms to which they are attached, form cycloalkyl or heterocyclyl;
m is an integer from 0 to 4;
n is 1 to 10, and n is an integer or decimal;
D is a camptothecin drug, preferably exatecan or a derivative thereof.

**2.** The antibody-drug conjugate or the pharmaceutically acceptable salt thereof according to claim 1, wherein the VH of the anti-c-Met antibody comprises a HCDR1, a HCDR2, and a HCDR3 set forth in SEQ ID NOs: 1-3, respectively, and the VL comprises a LCDR1, a LCDR2, and a LCDR3 set forth in SEQ ID NOs: 22, 5, and 6, respectively;

preferably, 1) the VH of the anti-c-Met antibody comprises a HCDR1, a HCDR2, and a HCDR3 set forth in SEQ ID NOs: 1, 2, and 3, respectively, and the VL comprises a LCDR1, a LCDR2, and a LCDR3 set forth in SEQ ID NOs: 7, 5, and 6, respectively;
or 2) the VH of the anti-c-Met antibody comprises a HCDR1, a HCDR2, and a HCDR3 set forth in SEQ ID NOs: 1, 2, and 3, respectively, and the VL comprises a LCDR1, a LCDR2, and a LCDR3 set forth in SEQ ID NOs: 4, 5, and 6, respectively;
the CDRs are defined according to the Kabat numbering scheme.

**3.** The antibody-drug conjugate or the pharmaceutically acceptable salt thereof according to claim 1 or 2, wherein the anti-c-Met antibody is a murine antibody, a chimeric antibody, a humanized antibody, or a fully human antibody, preferably a humanized antibody.

**4.** The antibody-drug conjugate or the pharmaceutically acceptable salt thereof according to claim 3, wherein the anti-c-Met antibody comprises any one of the following:

(1) the VH comprises the amino acid sequence set forth in SEQ ID NO: 11 or an amino acid sequence having at least 90% sequence identity thereto, and the VL comprises the amino acid sequence set forth in SEQ ID NO: 14 or an amino acid sequence having at least 90% sequence identity thereto;
(2) the VH comprises the amino acid sequence set forth in SEQ ID NO: 10 or an amino acid sequence having at least 90% sequence identity thereto, and the VL comprises the amino acid sequence set forth in SEQ ID NO: 13 or an amino acid sequence having at least 90% sequence identity thereto;
(3) the VH comprises the amino acid sequence set forth in SEQ ID NO: 12 or an amino acid sequence having at least 90% sequence identity thereto, and the VL comprises the amino acid sequence set forth in SEQ ID NO: 15 or an amino acid sequence having at least 90% sequence identity thereto.

**5.** The antibody-drug conjugate or the pharmaceutically acceptable salt thereof according to any one of claims 1-4, wherein the anti-c-Met antibody further comprises an Fc region; preferably, the Fc region is derived from the Fc region of human IgG1, IgG2, IgG3, or IgG4; more preferably, the Fc region is the Fc region of human IgG2.

**6.** The antibody-drug conjugate or the pharmaceutically acceptable salt thereof according to any one of claims 1-5, wherein the anti-c-Met antibody comprises a combination of a light chain and a heavy chain selected from the group consisting of the following:

the heavy chain comprises the amino acid sequence set forth in SEQ ID NO: 18 or an amino acid sequence having at least 90% sequence identity thereto, and the light chain comprises the amino acid sequence set forth in SEQ ID NO: 19 or an amino acid sequence having at least 90% sequence identity thereto;
the heavy chain comprises the amino acid sequence set forth in SEQ ID NO: 16 or an amino acid sequence having

at least 90% sequence identity thereto, and the light chain comprises the amino acid sequence set forth in SEQ ID NO: 17 or an amino acid sequence having at least 90% sequence identity thereto; and
the heavy chain comprises the amino acid sequence set forth in SEQ ID NO: 20 or an amino acid sequence having at least 90% sequence identity thereto, and the light chain comprises the amino acid sequence set forth in SEQ ID NO: 21 or an amino acid sequence having at least 90% sequence identity thereto.

**7.** The antibody-drug conjugate or the pharmaceutically acceptable salt thereof according to any one of claims 1-6, wherein n is 1 to 8, and n is a decimal or integer;

preferably, n is an integer or decimal from 3 to 7;
more preferably, n is an integer or decimal from 4 to 6.

**8.** The antibody-drug conjugate or the pharmaceutically acceptable salt thereof according to any one of claims 1-7, being an antibody-drug conjugate represented by formula II or a pharmaceutically acceptable salt thereof:

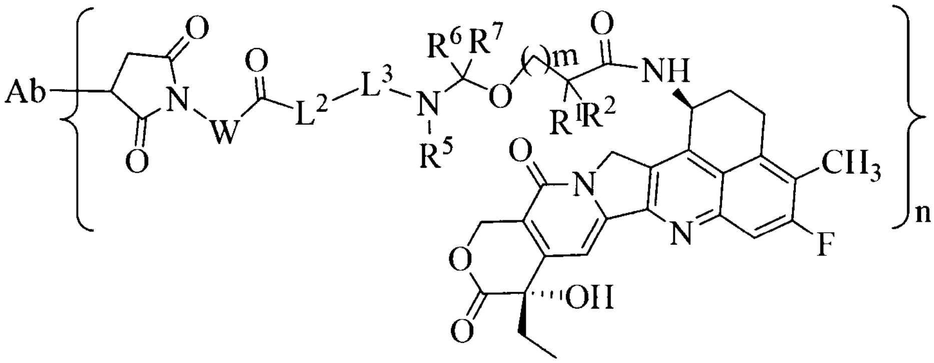

formula (II),

wherein:

W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;
$L^2$ is selected from the group consisting of $-NR^4(CH_2CH_2O)p^1CH_2CH_2C(O)-$, - $NR^4(CH_2CH_2O)p^1CH_2C(O)-$, $-S(CH_2)p^1C(O)-$, and a chemical bond, and $p^1$ is an integer from 1 to 20;
$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;
$R^1$ is selected from the group consisting of hydrogen, halogen, cycloalkylalkyl, deuterated alkyl, cycloalkyl, heterocyclyl, aryl, and heteroaryl;
$R^2$ is selected from the group consisting of hydrogen, halogen, haloalkyl, deuterated alkyl, cycloalkyl, heterocyclyl, aryl, and heteroaryl;
or $R^1$ and $R^2$, together with the carbon atom to which they are attached, form cycloalkyl or heterocyclyl;
$R^4$ and $R^5$ are identical or different and are each independently selected from the group consisting of hydrogen, alkyl, haloalkyl, deuterated alkyl, and hydroxyalkyl;
$R^6$ and $R^7$ are identical or different and are each independently selected from the group consisting of hydrogen, halogen, alkyl, haloalkyl, deuterated alkyl, and hydroxyalkyl;
m is an integer from 0 to 4;
n is 1 to 10, and n is an integer or decimal;
Ab is as defined in any one of claims 1-6.

**9.** The antibody-drug conjugate or the pharmaceutically acceptable salt thereof according to any one of claims 1-8, wherein:

the -L-Y- is represented by the following structure:

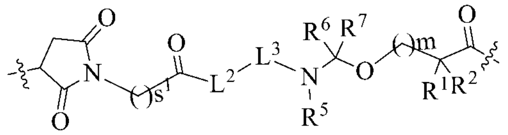

(-L-Y-) ;

$s^1$ is an integer from 2 to 8;
$L^2$, $L^3$, $R^1$, $R^2$, $R^5$, $R^6$, $R^7$, and m are as defined in claim 8.

**10.** The antibody-drug conjugate or the pharmaceutically acceptable salt thereof according to any one of claims 1-9, being an antibody-drug conjugate represented by general formula (III) or a pharmaceutically acceptable salt or solvate thereof:

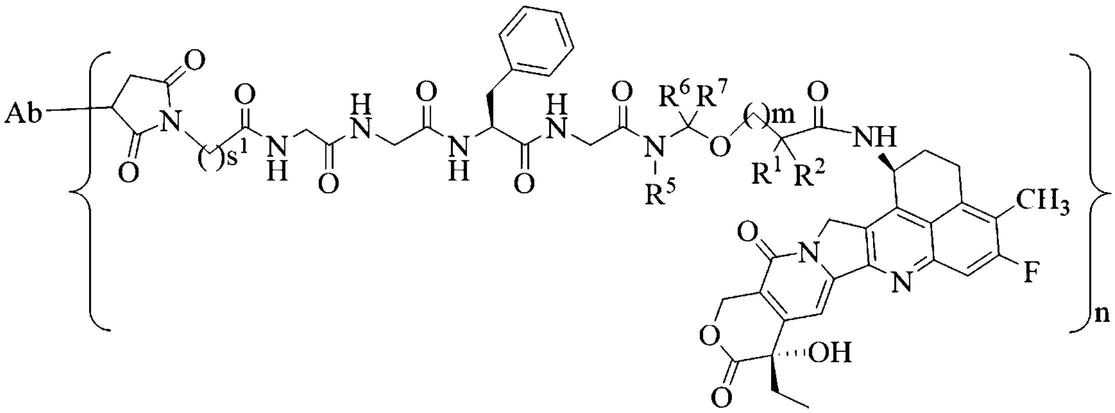

formula (III),

wherein:

$s^1$ is an integer from 2 to 8, preferably 5;
Ab, $R^1$, $R^2$, $R^5$, $R^6$, $R^7$, m, and n are as defined in claim 8.

**11.** The antibody-drug conjugate or the pharmaceutically acceptable salt thereof according to any one of claims 1-10, wherein -L- is:

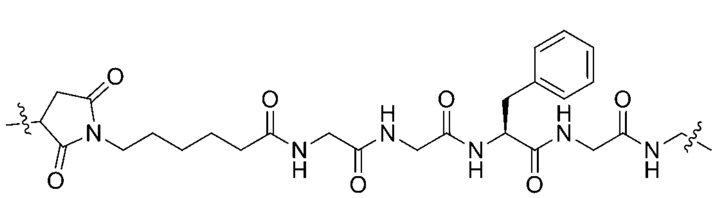

.

**12.** The antibody-drug conjugate or the pharmaceutically acceptable salt thereof according to any one of claims 1-11, wherein -L-Y- is selected from the group consisting of:

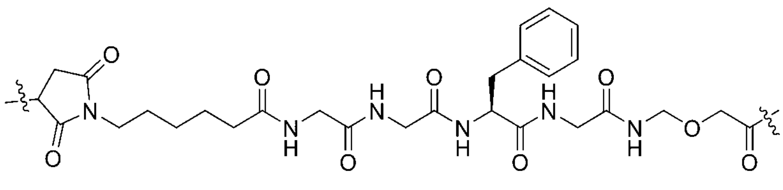

and

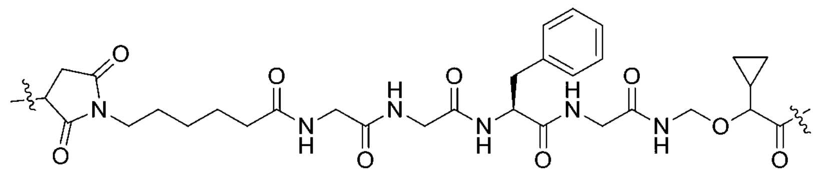

,

and is preferably

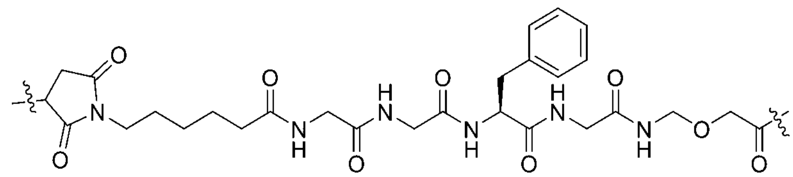

,

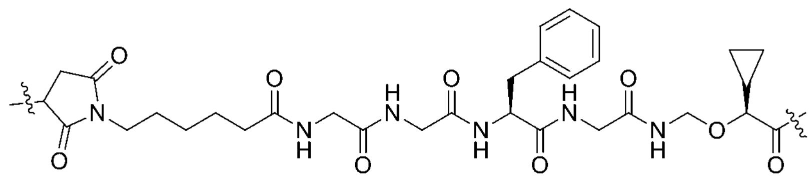

,

or

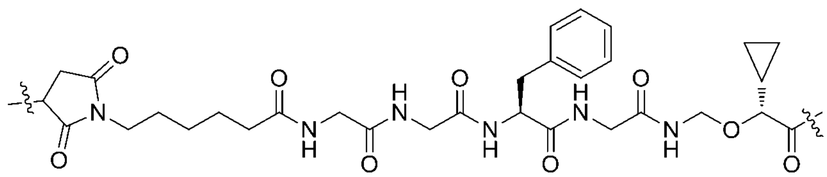

.

**13.** A method for preparing the antibody-drug conjugate or the pharmaceutically acceptable salt thereof according to any one of claims 1-12, comprising:

subjecting Ab and L-Y-D to a coupling reaction to give the antibody-drug conjugate,
wherein optionally, the method further comprises: purifying the antibody-drug conjugate.

**14.** A pharmaceutical composition, comprising:

the antibody-drug conjugate or the pharmaceutically acceptable salt thereof according to any one of claims 1-12, and
one or more pharmaceutically acceptable excipients, diluents, or carriers.

**15.** Use of the antibody-drug conjugate according to any one of claims 1-12 or the pharmaceutical composition according to claim 14 in the manufacture of a medicament for treating a disease or disorder, wherein:

preferably, the disease or disorder is a cancer or a c-Met-mediated disease or disorder;
more preferably, the cancer is a cancer expressing c-Met;
most preferably, the cancer is gastric cancer, lung cancer, intestinal cancer, or liver cancer.

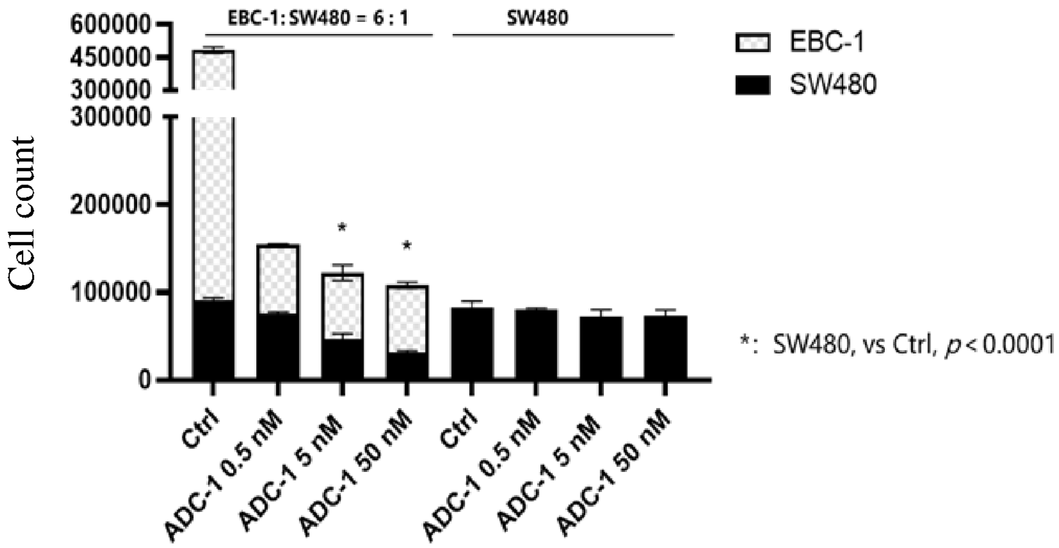
EBC-1: SW480 = 6 : 1
SW480
EBC-1
SW480
Cell count
600000
450000
300000
300000
200000
100000
0
*
*
*: SW480, vs Ctrl, p < 0.0001
Ctrl
ADC-1 0.5 nM
ADC-1 5 nM
ADC-1 50 nM
Ctrl
ADC-1 0.5 nM
ADC-1 5 nM
ADC-1 50 nM

**FIG. 1**

**INTERNATIONAL SEARCH REPORT**

International application No.
**PCT/CN2024/107536**

**A. CLASSIFICATION OF SUBJECT MATTER**

A61K 47/68(2017.01)i； A61K31/506(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： A61K， A61P

Documentation searched other than minimum documentation to the extent that such documents are included in the fields searched

Electronic data base consulted during the international search (name of data base and, where practicable, search terms used)

WPABSC, CNTXT, CNABS, WPABS, DWPI, ENTXTC, ISI_Web of Science, STNext, CNKI, 万方, WANFANG: 江苏恒瑞医药股份有限公司, 上海盛迪医药有限公司, 抗c-Met抗体, 喜树碱, 依喜替康, 艾沙替康, 依沙替康, exatecan, 偶联物, 缀合, conjugation, coupling, linker, ADC, antibody drug conjugate

**C. DOCUMENTS CONSIDERED TO BE RELEVANT**

| Category* | Citation of document, with indication, where appropriate, of the relevant passages | Relevant to claim No. |
|---|---|---|
| Y | CN 106188293 A (JIANGSU HENGRUI PHARMACEUTICALS CO., LTD. et al.) 07 December 2016 (2016-12-07)<br>abstract, claims 26, 33, and 40-42, and description, paragraphs 323-368, sequences 6-18 and 23-28, and paragraph 137 | 1-15 |
| Y | WO 2021190583 A1 (JIANGSU HENGRUI PHARMACEUTICALS CO., LTD. et al.) 30 September 2021 (2021-09-30)<br>claims 1, 9, 11-12, and 15 | 1-15 |
| Y | WO 2016165580 A1 (JIANGSU HENGRUI PHARMACEUTICALS CO., LTD. et al.) 20 October 2016 (2016-10-20)<br>claims 1-42 | 1-15 |
| Y | WO 2021190564 A1 (SHANGHAI HANSOH BIOMEDICAL CO., LTD. et al.) 30 September 2021 (2021-09-30)<br>claims 1, 9-11, and 16 | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 October 2024** | **17 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

INTERNATIONAL SEARCH REPORT

International application No.
PCT/CN2024/107536

C. DOCUMENTS CONSIDERED TO BE RELEVANT

| Category* | Citation of document, with indication, where appropriate, of the relevant passages | Relevant to claim No. |
|---|---|---|
| Y | WO 2021190480 A1 (SHANGHAI HANSOH BIOMEDICAL CO., LTD. et al.) 30 September 2021 (2021-09-30)<br>claims 1-14 | 1-15 |
| Y | US 2017348429 A1 (ABBVIE BIOTHERAPEUTICS INC.) 07 December 2017 (2017-12-07)<br>claims 1-39 | 1-15 |

Form PCT/ISA/210 (second sheet) (July 2022)

**INTERNATIONAL SEARCH REPORT**

International application No.
**PCT/CN2024/107536**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/107536**

| Patent document cited in search report | | | Publication date (day/month/year) | Patent family member(s) | | | Publication date (day/month/year) |
|---|---|---|---|---|---|---|---|
| CN | 106188293 | A | 07 December 2016 | BR | 112017021245 | A2 | 26 June 2018 |
| | | | | JP | 2018516539 | A | 28 June 2018 |
| | | | | CA | 2982777 | A1 | 20 October 2016 |
| | | | | KR | 20170138451 | A | 15 December 2017 |
| | | | | MX | 2017012965 | A | 06 June 2018 |
| | | | | RU | 2017135257 | A | 17 May 2019 |
| | | | | RU | 2017135257 | A3 | 24 September 2019 |
| | | | | US | 2018110875 | A1 | 26 April 2018 |
| | | | | US | 10543284 | B2 | 28 January 2020 |
| | | | | EP | 3284751 | A1 | 21 February 2018 |
| | | | | EP | 3284751 | A4 | 05 December 2018 |
| | | | | TW | 201638108 | A | 01 November 2016 |
| | | | | TWI | 731856 | B | 01 July 2021 |
| | | | | AU | 2016248357 | A1 | 26 October 2017 |
| WO | 2021190583 | A1 | 30 September 2021 | JP | 2023519261 | A | 10 May 2023 |
| | | | | TW | 202144016 | A | 01 December 2021 |
| | | | | EP | 4130006 | A1 | 08 February 2023 |
| | | | | EP | 4130006 | A4 | 17 January 2024 |
| | | | | BR | 112022019027 | A2 | 01 November 2022 |
| | | | | US | 2023140397 | A1 | 04 May 2023 |
| | | | | CA | 3177279 | A1 | 30 September 2021 |
| | | | | KR | 20220160016 | A | 05 December 2022 |
| | | | | ZA | 202210724 | B | 20 December 2023 |
| | | | | AU | 2021240756 | A1 | 17 November 2022 |
| | | | | MX | 2022011808 | A | 06 December 2022 |
| WO | 2016165580 | A1 | 20 October 2016 | None | | | |
| WO | 2021190564 | A1 | 30 September 2021 | TW | 202144017 | A | 01 December 2021 |
| WO | 2021190480 | A1 | 30 September 2021 | TW | 202144012 | A | 01 December 2021 |
| US | 2017348429 | A1 | 07 December 2017 | CY | 1123858 | T1 | 27 May 2022 |
| | | | | PT | 3458102 | T | 17 August 2020 |
| | | | | HRP | 20210326 | T1 | 02 April 2021 |
| | | | | LT | 3458102 | T | 25 August 2020 |
| | | | | AU | 2017268342 | A1 | 29 November 2018 |
| | | | | AU | 2017268342 | B2 | 17 August 2023 |
| | | | | SG | 10201914095 | SA | 30 March 2020 |
| | | | | HUE | 053332 | T2 | 28 June 2021 |
| | | | | RS | 61659 | B1 | 29 April 2021 |
| | | | | SI | 3626273 | T1 | 30 April 2021 |
| | | | | RU | 2018144315 | A | 17 June 2020 |
| | | | | RU | 2018144315 | A3 | 22 July 2020 |
| | | | | RU | 2740996 | C2 | 22 January 2021 |
| | | | | HUE | 050398 | T2 | 28 December 2020 |
| | | | | KR | 20230028574 | A | 28 February 2023 |
| | | | | LT | 3626273 | T | 10 March 2021 |
| | | | | SG | 11201810034 | XA | 28 December 2018 |
| | | | | MX | 2018014175 | A | 07 February 2020 |
| | | | | JP | 6870161 | B1 | 12 May 2021 |
| | | | | JP | 2021073247 | A | 13 May 2021 |
| | | | | ZA | 201808386 | B | 27 July 2022 |

Form PCT/ISA/210 (patent family annex) (July 2022)

**INTERNATIONAL SEARCH REPORT**
**Information on patent family members**

International application No.
**PCT/CN2024/107536**

| Patent document cited in search report | Publication date (day/month/year) | Patent family member(s) | | | Publication date (day/month/year) |
|---|---|---|---|---|---|
| | | IL | 294146 | A | 01 August 2022 |
| | | IL | 294146 | B1 | 01 September 2023 |
| | | IL | 294146 | B2 | 01 January 2024 |
| | | BR | 112018073574 | A2 | 19 March 2019 |
| | | KR | 20190008339 | A | 23 January 2019 |
| | | KR | 102449294 | B1 | 30 September 2022 |
| | | ES | 2811351 | T3 | 11 March 2021 |
| | | EP | 3458102 | A1 | 27 March 2019 |
| | | EP | 3458102 | B1 | 17 June 2020 |
| | | JP | 2019521087 | A | 25 July 2019 |
| | | KR | 20220137158 | A | 11 October 2022 |
| | | KR | 102512597 | B1 | 23 March 2023 |
| | | RS | 60663 | B1 | 30 September 2020 |
| | | SI | 3458102 | T1 | 30 September 2020 |
| | | US | 2023321271 | A1 | 12 October 2023 |
| | | US | 2018250418 | A1 | 06 September 2018 |
| | | US | 10383948 | B2 | 20 August 2019 |
| | | DK | 3458102 | T3 | 27 July 2020 |
| | | EP | 4233909 | A2 | 30 August 2023 |
| | | EP | 4233909 | A3 | 20 September 2023 |
| | | EP | 3804765 | A1 | 14 April 2021 |
| | | US | 2019314518 | A1 | 17 October 2019 |
| | | US | 10603389 | B2 | 31 March 2020 |
| | | JP | 2022185003 | A | 13 December 2022 |
| | | PL | 3458102 | T3 | 14 December 2020 |
| | | HRP | 20201186 | T1 | 13 November 2020 |
| | | US | 2020215200 | A1 | 09 July 2020 |
| | | JP | 2021107424 | A | 29 July 2021 |
| | | JP | 7150087 | B2 | 07 October 2022 |
| | | DK | 3626273 | T3 | 08 February 2021 |
| | | CY | 1123256 | T1 | 29 October 2021 |
| | | PL | 3626273 | T3 | 14 June 2021 |
| | | CA | 3024386 | A1 | 23 November 2017 |
| | | CA | 3024386 | C | 02 January 2024 |
| | | WO | 2017201204 | A1 | 23 November 2017 |
| | | NZ | 748314 | A | 28 July 2023 |
| | | IL | 304717 | A | 01 September 2023 |
| | | EP | 3626273 | A1 | 25 March 2020 |
| | | EP | 3626273 | B1 | 30 December 2020 |
| | | PT | 3626273 | T | 09 March 2021 |
| | | ES | 2864150 | T3 | 13 October 2021 |
| | | IL | 262869 | A | 31 December 2018 |
| | | IL | 262869 | B | 01 July 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310916920
- WO 2017201204 A
- WO 2018142322 A
- CN 104755494 A
- WO 2013106717 A
- CN 105829346 A
- EP 2907824 A
- DD 110102
- CN 106687480 B


### Non-patent literature cited in the description


- *J. biol. chem.*, 1968, vol. 243, 3558
- **KABAT, E. A. et al.** Sequences of Proteins of Immunological Interest. 1991
- **LI N. et al.** *Methods Mol. Biol.*, 2008, vol. 457, 305-17
- **PAHARA J. et al.** *Exp Cell Res.*, 2010, vol. 316, 2237-50
- **MAZOR et al.** *J. Immunol. Methods*, 2007, vol. 321, 41-59
- Chinese Pharmacopoeia. 2015, vol. 0542
- Antibodies: A Laboratory Manual and Molecular Cloning: A Laboratory Manual. Cold Spring Harbor Laboratory
- *WHO Drug Information*, 2016, vol. 30 (2)